# EUROPEAN PATENT SPECIFICATION

(11) **EP 1 246 933 B1**
(45) Date of publication and mention of the grant of the patent: **21.07.2010**
(21) Application number: 00983074.6
(22) Date of filing: 04.12.2000
(51) Int. Cl.: C12P 21/02, C07K 14/62

(54) **METHOD FOR MAKING INSULIN PRECURSORS AND INSULIN PRECURSOR ANALOGUES HAVING IMPROVED FERMENTATION YIELD IN YEAST**
VERFAHREN ZUR HERSTELLUNG VON INSULINVORLäUFERN UND ANALOGEN VON INSULINVORLÄUFERN MIT VERBESSERTER FERMENTATIONSAUSBEUTE IN HEFE
PROCEDE DE FABRICATION DE PRECURSEURS D'INSULINE ET ANALOGUES DES PRECURSEURS D'INSULINE PRESENTANT UN MEILLEUR RENDEMENT DE FERMENTATION DANS LA LEVURE

(30) Priority: 29.12.1999 DK 186999; 17.03.2000 DK 200000443
(43) Date of publication of application: 09.10.2002
(73) Proprietor: NOVO NORDISK A/S, 2880 Bagsværd (DK)
(72) Inventor: KJELDSEN, Thomas, Borglum, DK-2830 Virum (DK); LUDVIGSEN, Svend, DK-3540 Lynge (DK)
(74) Representative: Secher, Anne
(86) International application number: PCT/DK2000/000664
(87) International publication number: WO 2001/049870

(56) References cited:
- EP-A2- 0 055 945
- EP-A2- 0 741 188
- US-A- 5 962 267
- SEUNG-GU CHANG ET AL.: 'Human insulin production from a novel mini-proinsulin which has high receptor-binding activity' BIOCHEM. J., [Online] vol. 329, 1998, pages 631 - 635, XP002938394 Retrieved from the Internet: <URL:http://www.biochemj.org/bj/329/0631/bj 3290631.htm> [retrieved on 2001-04-06]
- RONALD WETZEL ET AL.: 'Expression in escherichia coli of a chemically synthesized gene for a mini-C analog of human proinsulin' GENE vol. 16, 1981, pages 63 - 71, XP002938395

## Description

### BACKGROUND

Yeast organisms produce a number of proteins that have a function outside the cell. Such proteins are referred to as secreted proteins. These secreted proteins are expressed initially inside the cell in a precursor or a pre-form containing a pre-peptide sequence ensuring effective direction (translocation) of the expressed product across the membrane of the endoplasmic reticulum (ER). The pre-peptide, normally named a signal peptide, is generally cleaved off from the desired product during translocation. Once entered in the secretory pathway, the protein is transported to the Golgi apparatus. From the Golgi, the protein can follow different routes that lead to compartments such as the cell vacuole or the cell membrane, or it can be routed out of the cell to be secreted to the external medium (Pfeffer et al. (1987) Ann. Rev. Biochem. 56:829-852).

Insulin is a polypeptide hormone secreted by β-cells of the pancreas and consists of two polypeptide chains, A and B, which are linked by two inter-chain disulphide bridges. Furthermore, the A-chain features one intra-chain disulphide bridge.

The hormone is synthesized as a single-chain precursor proinsulin (preproinsulin) consisting of a prepeptide of 24 amino acid followed by proinsulin containing 86 amino acids, in the configuration: prepeptide - B - Arg Arg - C - Lys Arg -A, in which C is a connecting peptide of 31 amino acids. Arg-Arg and Lys-Arg are cleavage sites for cleavage of the connecting peptide from the A and B chains.

Three major methods have been used for the production of human insulin in microorganisms. Two involve *Escherichia coli,* with either the expression of a large fusion protein in the cytoplasm (Frank et al. (1981) in Peptides: Proceedings of the 7th American Peptide Chemistry Symposium (Rich & Gross, eds.), Pierce Chemical Co., Rockford, IL. pp 729-739), or use a signal peptide to enable secretion into the periplasmic space (Chan et al. (1981) PNAS 78:5401-5404). A third method utilizes *Saccharomyces cerevisiae* to secrete an insulin precursor into the medium (Thim et al. (1986) PNAS 83:6766-6770). The prior art discloses a limited number of insulin precursors which are expressed in either *E. coli* or Sac*charomyces cerevisiae,* vide US 5,962,267, WO 95/16708, EP 0055945, EP 0163529, EP 0347845 and EP 0741188.

### SUMMARY OF THE INVENTION

The present invention features novel connecting peptides (mini C-peptides) which confer an increased production yield in insulin precursor molecules and insulin precursor analog molecules when expressed in a transformed microorganism, in particular in yeast. Such insulin precursors or insulin precursor analogs can then be converted into insulin or insulin analogs by one or more suitable, well known conversion steps.

The connecting peptides of the present invention contain one Gly and will be shorter than the natural human C peptide which, including the flanking dibasic cleavage sites, consists of 35 amino acids. Thus the novel connecting peptides will in general not be of more than 4 amino acid residues in length.

As in the natural human insulin molecule, the connecting peptide will contain a cleavage site at its C and N termini enabling in vitro cleavage of the connecting peptide from the A and B chains. Such cleavage sites may be any convenient cleavage sites known in the art, e.g. a Met cleavable by cyanogen bromide; a single basic amino acid residue or a pair of basic amino acid residues (Lys or Arg) cleavable by trypsin or trypsin like proteases; *Acromobactor lyticus* protease or by a carboxypeptidase protease. The cleavage site enabling cleavage of the connecting peptide from the A-chain is a single basic amino acid residue Lys or Arg, preferably Lys.

Alternatively cleavage of the connecting peptide from the B chain may be enabled by cleavage at the natural Lys^{B29} amino acid residue in the B chain giving rise to a desB30 insulin precursor or desB30 insulin precursor analog. The desired B30 amino acid residue may then be added by well known in vitro, enzymatic procedures.

In one embodiment the connecting peptide will not contain two adjacent basic amino acid residues (Lys,Arg). In this embodiment, cleavage from the A-chain may be accomplished at a single Lys or Arg located at the N-terminal end of the A-chain and the natural Lys in position B29 in the B-chain.

Furthermore, the B27 (atom CG2) will typically have a proximity to the A1 (atom CA) of less than 5 Å.

In one embodiment, the present invention is related to insulin precursors or insulin precursor analogs comprising a sequence of formula:

B(1-27)-X₃-X₂-X₁-Y-A(1-21),

wherein
X₁ comprises 1 - 3 amino acid residues in.length comprising a single Gly, immediately N-terminal to Y,
X₂ is one of Pro, Lys, Ala, Arg or Pro-Thr at position 29 of the B chain,
X₃ is one of Pro, Asp, Lys, or Ile at position 28 of the B chain, and
Y is Lys or Arg.

In a further embodiment X₁ is 1-2 amino acid residues in length.

The amino acid residues in X₁ can be any codable amino acid residue and may be the same or different with the only proviso that at least one amino acid residue in X₁ is Gly.

In one embodiment, X₃ is Asp and X₂ is Lys. This embodiment encompasses the insulin precursor analogs containing an Asp in position B28 of the B chain (termed hereinafter "Asp^{B28}IP"). In another embodiment X₂ is Lys and X₃ is Pro. In a further embodiment the sequence X₁- Y is selected from the group of: (a) Glu-Glu-Gly-Lys(SEQ ID NO:1, (b) Glu-Gly-Lys, (c) Ser-Gly-Lys, (d) Asn-Gly-Lys, (e) Thr-Gly-Lys, (f) Asp-Gly-Lys, (g) Met-Gly-Lys, (h) Ala-Gly-Lys, (i) His-Gly-Lys and (j) Gly-Lys.

In still further specific embodiments, X₁ is 1-3 amino acid residues; X₃ is Lys and X₂ is Pro. In a further embodiment, X₁ is 1-3 amino acid residues, X₃ is Asp and X₂ is Lys. In another embodiment X₂ is Pro, X₃ is Lys and X₁ is 1-2 amino acid residues of which one is Trp or Phe.

In a specific embodiment, the mini C-peptide comprises the sequence Glu-Gly-Lys, Asn-Gly-Lys, or Asp-Gly-Lys.

The present invention is also related to polynucleotide sequences which code for the claimed insulin precursors or insulin precursor analogs. In a further aspect the present invention is related to vectors containing such polynucleotide sequences and to host cells containing such polynucleotide sequences or vectors.

In another aspect, the invention relates to a process for producing the insulin precursors or insulin precursor analogs in a host cell, said method comprising (i) culturing a host cell comprising a polynucleotide sequence encoding the insulin precursors or insulin precursor analogs of the invention under suitable conditions for expression of said precursor or precursor analog; and (ii) isolating the precursor or precursor analog from the culture medium.

In still a further aspect, the invention relates to a process for producing insulin or insulin analogs in a host cell, said method comprising (i) culturing a host cell comprising a polynucleotide sequence encoding an insulin precursor or insulin precursor analogs of the invention; (ii) isolating the precursor or precursor analog from the culture medium and (iii) converting the precursor or precursor analog into insulin or an insulin analog by in vitro enzymatic conversion.

In one embodiment of the present invention the host cell is a yeast host cell and in a further embodiment the yeast host cell is selected from to the genus *Saccharomyces.* In a further embodiment the yeast host cell is selected from the species *Saccharomyces cerevisiae.*

In a related aspect, the invention features a mini C-peptide in an insulin precursor or insulin precursor analog wherein the amino acid residues of the C-peptide exhibit sufficient flexibility to allow several geometric arrangements of the C-peptide to accommodate an atomic distance between B27 CG2 and A1 CA less than 5 Å.

### BRIEF DESCRIPTION OF THE DRAWINGS

Fig. 1 represents the pAK721 *S. cerevisiae* expression plasmid expressing the LA19 leader- EEAEAEAEPK(SEQ ID NO:2)-IP(AlaAlaLys) fusion protein.
Fig. 2 is the DNA sequence and inferred amino acid sequence of the encoded fusion protein (α-factor-leader-EEAEAEAPK(SEQ ID NO:3)- Asp^{B28}IP portion of pAK1150 used as PCR template (SEQ ID NO:4 and 5).
Fig.3 is the DNA sequence encoding a leader-Asp^{B28}IP fusion protein with a synthetic mini C-peptide (DGK or AspGlyLys) generated by randomized optimization (SEQ ID NO:6 and 7). The mini C-peptide (DGK) is indicated by underlining.
Fig. 4 shows the solution structures of Asp^{B28}IP(AspGlyLys) as backbone lines of ensemble of 20 converged structures.
Fig.5 shows a ribbon presentation of Asp^{B28}IP(AspGlyLys). The figure is produced using MOLSCRIPT (Kraulis (1991) J. Appl. Crystallog. 24:946-950). Amino acid residue annotation is derived as follows: B1-B29 (B chain) are numbered 1-29, residues C1-C3 (C chain) are numbered 30-32, and residues A1-A21 (A chain) are numbered 33-53.

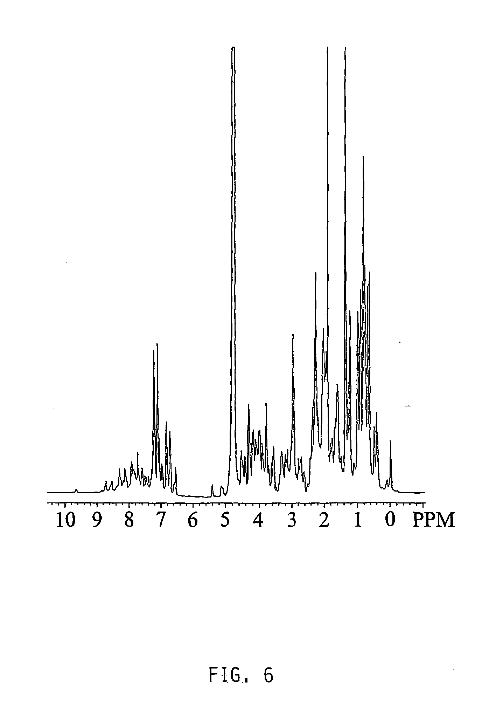

Fig. 6 is the ID proton NMR spectrum for Asp^{B28}IP(Asp Gly Lys) recorded at 27°C at 1.0 mM concentration in 10%/90% D₂O/H₂O with 10 mM phosphate buffer at pH 8.0.
Fig. 7 is DNA and inferred amino acid sequence of the expression cassette expressing the YAP3-TA39-GluGluGlyGluProLys(SEQ ID NO:8)-Asp^{B28}IP fusion protein with a synthetic mini C-peptide (DGK or AspGlyLys) (SEQ ID NO:9 and 10).
Fig. 8 is DNA and inferred amino acids sequences of the expression cassette expressing the YAP3-TA57-GluGluGlyGluProLys(SEQ ID NO:8)-Asp^{B28}IP fusion protein with a synthetic mini C-peptide (DGK or AspGlyLys) (SEQ ID NOS: 11 and 12).

### DETAILED DESCRIPTION

### Abbreviations and nomenclature.

By **"connecting peptide"** or **"C-peptide"** is meant the connection moiety **"C"** of the B-C-A polypeptide sequence of a single chain preproinsulin-like molecule. Specifically, in the natural insulin chain, the C-peptide connects position 30 of the B chain and position 1 of the A chain. A "mini C-peptide" or "connecting peptide" such as those described herein, connect B29 or B30 to A1, and differ in sequence and length from that of the natural C-peptide.

By **"IP"** is meant a single-chain insulin precursor in which a desB30 chain is linked to the A chain of insulin via a connecting peptide. The single-chain insulin precursor will contain correctly positioned disulphide bridges (three) as in human insulin.

With "desB30" or "B(1-29)" is meant a natural insulin B chain lacking the B30 amino acid residue, "A(1-21)" means the natural insulin A chain, "B(1-27)" means the natural B chain lacking the B28, B29, and B30 amino acid residues; "Asp^{B28}IP" means a single-chain insulin precursor with aspartic acid at position 28 of the B-chain and no C-peptide (B29 is linked to A1). The mini C-peptide and its amino acid sequence is indicated in the three letter amino acid code in parenthesis following the IP; Thus "Asp^{B28}IP(MetTrpLys)" means a single-chain insulin precursor with aspartic acid at position 28 of the B-chain and a mini C-peptide with the sequence Met-Trp-Lys connecting B29 to A1.

By "insulin **precursor"** is meant a single-chain polypeptide which by one or more subsequent chemical and/or enzymatic processes can be converted into human insulin.

By "insulin **precursor analog"** is meant an insulin precursor molecule having one or more mutations, substitutions, deletions and or additions of the A and/or B amino acid chains relative to the human insulin molecule. The insulin analogs are preferably such wherein one or more of the naturally occurring amino acid residues, preferably one, two, or three of them, have been substituted by another codable amino acid residue. In one embodiment, the instant invention comprises analog molecules having position 28 of the B chain altered relative to the natural human insulin molecule. In this embodiment, position 28 is modified from the natural Pro residue to one of Asp, Lys, or Ile. In a preferred embodiment, the natural Pro residue at position B28 is modified to an Asp residue. In another embodiment Lys at position B29 is modified to Pro; Also, Asn at position A21 may be modified to Ala, Gln, Glu, Gly, His, Ile, Leu, Met, Ser, Thr, Trp, Tyr or Val, in particular to Gly, Ala, Ser, or Thr and preferably to Gly. Furthermore, Asn at position B3 may be modified to Lys. Further examples of insulin precursor analogs are des(B30) human insulin, insulin analogs wherein Phe^{B1} has been deleted; insulin analogs wherein the A-chain and/or the B-chain have an N-terminal extension and insulin analogs wherein the A-chain and/or the B-chain have a C-terminal extension. Thus one or two Arg may be added to position B1.

The term **"immediately N-terminal to"** is meant to illustrate, the situation where an amino acid residue or a peptide sequence is directly linked at its C-terminal end to the N-terminal end of another amino acid residue or amino acid sequence by means of a peptide bond.

In the present context, the term **"functional analog of insulin"** and the like, is meant to indicate a polypeptide with a similar biological action as the native human insulin protein.

By a distance **shorter than 5** Å between two amino acid residues is meant the shortest inter-atomic distance less than 5 Å between any atom in the first amino acid and any atom in the second amino acid. Atomic distances are measured from three-dimensional structures of the molecule determined either by NMR (Wüthrich, K., 1986, NMR of Proteins and Nucleic Acids, Wiley, New York) or by X-ray crystallography (Drenth, J., 1994, Principles of Protein X-ray crystallography, Springer Verlag Berlin). A distance from one amino acid to another is measured as the shortest inter-atomic distance between any atom in the first amino acid and any atom in the second amino acid if not stated differently.

The present invention features novel mini C-peptides connecting position 29 of the insulin B chain and position 1 of the insulin A chain which significantly increased production yields in a yeast host cell. By the term **"significantly increased production," "increased fermentation yield,"** and the like, is meant an increase in secreted amount of the insulin precursor molecule or insulin precursor analog molecule present in the culture supernatant compared to the yield of an insulin precursor or insulin precursor analog with no aromatic amino acid residue in the mini C peptide. An **"increased"** fermentation yield is an absolute number larger than the control; preferably, the increase is 50% or more larger than the control (AspB²⁸ IP) level; even more preferably, the increase is 100% or more larger than control levels.

**"*POT*"** is the *Schizosaccharomyces pombe* triose phosphate isomerase gene, and **"*TPI1*"** is the *S. cerevisiae* triose phosphate isomerase gene.

By a **"leader"** is meant an amino acid sequence consisting of a pre-peptide (the signal peptide) and a pro-peptide.

The term **"signal peptide"** is understood to mean a pre-peptide which is present as an N-terminal sequence on the precursor form of a protein. The function of the signal peptide is to allow the heterologous protein to facilitate translocation into the endoplasmic reticulum. The signal peptide is normally cleaved off in the course of this process. The signal peptide may be heterologous or homologous to the yeast organism producing the protein. A number of signal peptides which may be used with the DNA construct of the invention including yeast aspartic protease 3 (YAP3) signal peptide or any functional analog (Egel-Mitani et al. (1990) YEAST 6:127-137 and US 5,726,038) and the α-factor signal of the *MFα1* gene (Thorner (1981) in The Molecular Biology of the Yeast Saccharomyces cerevisiae, Strathern et al., eds., pp 143-180, Cold Spring Harbor Laboratory, NY and US 4,870,00.

The term **"pro-peptide"** means a polypeptide sequence whose function is to allow the expressed polypeptide to be directed from the endoplasmic reticulum to the Golgi apparatus and further to a secretory vesicle for secretion into the culture medium (i.e. exportation of the polypeptide across the cell wall or at least through the cellular membrane into the periplasmic space of the yeast cell). The pro-peptide may be the yeast α-factor pro-peptide, vide US 4,546,082 and 4,870,008. Alternatively, the pro-peptide may be a synthetic pro-peptide, which is to say a pro-peptide not found in nature. Suitable synthetic pro-peptides are those disclosed in US 5,395,922; 5,795,746; 5,162,498 and WO 98/32867. The pro- peptide will preferably contain an endopeptidase processing site at the C-terminal end, such as a Lys-Arg sequence or any functional analog thereof.

The polynucleotide sequence of the invention may be prepared synthetically by established standard methods, e.g. the phosphoamidite method described by Beaucage et al. (1981) Tetrahedron Letters 22:1859-1869, or the method described by Matthes et al. (1984) EMBO Journal 3:801-805. According to the phosphoamidite method, oligonucleotides are synthesized, for example, in an automatic DNA synthesizer, purified, duplexed and ligated to form the synthetic DNA construct. A currently preferred way of preparing the DNA construct is by polymerase chain reaction (PCR).

The polynucleotide sequence of the invention may also be of mixed genomic, cDNA, and synthetic origin. For example, a genomic or cDNA sequence encoding a leader peptide may be joined to a genomic or cDNA sequence encoding the A and B chains, after which the DNA sequence may be modified at a site by inserting synthetic oligonucleotides encoding the desired amino acid sequence for homologous recombination in accordance with well-known procedures or preferably generating the desired sequence by PCR using suitable oligonucleotides.

The invention encompasses a vector which is capable of replicating in the selected microorganism or cell line and which carries a polynucleotide sequence encoding the insulin precursors or insulin precursor analogs of the invention. The recombinant vector may be an autonomously replicating vector, *i.e*., a vector which exists as an extra-chromosomal entity, the replication of which is independent of chromosomal replication, *e.g*., a plasmid, an extra-chromosomal element, a mini-chromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used. The vectors may be linear or closed circular plasmids and will preferably contain an element(s) that permits stable integration of the vector into the host cell's genome or autonomous replication of the vector in the cell independent of the genome.

In a preferred embodiment, the recombinant expression vector is capable of replicating in yeast organisms. Examples of sequences which enable the vector to replicate in yeast are the yeast plasmid 2 µm replication genes REP 1-3 and origin of replication.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the *dal* genes from *Bacillus subtilis* or *Bacillus licheniformis,* or markers which confer antibiotic resistance such as ampicillin, kanamycin, chloramphenicol or tetracycline resistance. Selectable markers for use in a filamentous fungal host cell include *amdS* (acetamidase), *argB* (ornithine carbamoyl-transferase), *pyrG* (orotidine-5'-phosphate decarboxylase), *sC* (sulfate adenyltransferase) and *trpC* (anthranilate synthase. Suitable markers for yeast host cells are ADE2, HIS3, LEU2, LYS2, MET3, TRP1, and URA3. A preferred selectable marker for yeast is the *Schizosaccharomyces pompe* TPI gene (Russell (1985) Gene 40:125-130).

In the vector, the polynucleotide sequence is operably connected to a suitable promoter sequence. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription in a bacterial host cell, are the promoters obtained from the *E. coli lac* operon, *Streptomyces coelicolor* agarase gene (*dagA*), *Bacillus subtilis* levansucrase gene (*sacB*), *Bacillus licheniformis* alpha-amylase gene (*amyL*), *Bacillus stearothermophilus* maltogenic amylase gene (*amyM*), *Bacillus amyloliquefaciens* alpha-amylase gene (*amyQ*), and *Bacillus licheniformis* penicillinase gene (*penP*). Examples of suitable promoters for directing the transcription in a filamentous fungal host cell are promoters obtained from the genes for *Aspergillus oryzae* TAKA amylase, *Rhizomucor miehei* aspartic proteinase, *Aspergillus niger* neutral alpha-amylase, and *Aspergillus niger* acid stable alpha-amylase. In a yeast host, useful promoters are the *Saccharomyces cerevisiae* Ma1, TPI, ADH or PGK promoters.

The polynucleotide construct of the invention will also typically be operably connected to a suitable terminator. In yeast a suitable terminator is the TPI terminator (Alber et al. (1982) J. Mol. Appl. Genet. 1:419-434).

The procedures used to ligate the polynucleotide sequence of the invention, the promoter and the terminator, respectively, and to insert them into suitable yeast vectors containing the information necessary for yeast replication, are well known to persons skilled in the art. It will be understood that the vector may be constructed either by first preparing a DNA construct containing the entire DNA sequence of the invention, and subsequently inserting this fragment into a suitable expression vector, or by sequentially inserting DNA fragments containing genetic information for the individual elements (such as the signal, pro-peptide, mini C-peptide, A and B chains) followed by ligation.

The present invention also relates to recombinant host cells, comprising a polynucleotide sequence encoding the insulin precursors or the insulin precursor analogs of the invention. A vector comprising such polynucleotide sequence is introduced into the host cell so that the vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source. The host cell may be a unicellular microorganism, *e.g*., a prokaryote, or a non-unicellular microorganism, *e.g*., a eukaryote. Useful unicellular cells are bacterial cells such as gram positive bacteria including, but not limited to, a *Bacillus* cell, *Streptomyces* cell, or gram negative bacteria such as *E. coli* and *Pseudomonas* sp. Eukaryote cells may be mammalian, insect, plant, or fungal cells. In a preferred embodiment, the host cell is a yeast cell. The yeast organism used in the process of the invention may be any suitable yeast organism which, on cultivation, produces large amounts of the insulin precursor and insulin precursor analogs of the invention. Examples of suitable yeast organisms are strains selected from the yeast species *Saccharomyces cerevisiae, Saccharomyces kluyveri, Schizosaccharomyces pombe, Sacchoromyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp.,* and *Geotrichum fermentans.*

The transformation of the yeast cells may for instance be effected by protoplast formation followed by transformation in a manner known *per se.* The medium used to cultivate the cells may be any conventional medium suitable for growing yeast organisms. The secreted insulin precursor or insulin precursor analogs of the invention, a significant proportion of which will be present in the medium in correctly processed form, may be recovered from the medium by conventional procedures including separating the yeast cells from the medium by centrifugation, filtration or catching the insulin precursor or insulin precursor analog by an ion exchange matrix or by a reverse phase absorption matrix, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

The insulin precursors and insulin precursor analogs of the invention may be expressed with an N-terminal amino acid residue extension, as described in U.S. Patent No. 5,395,922, and European Patent No. 765,395A, both of which patents are herein specifically incorporated by reference. The extension is found to be stably attached to the insulin precursor or insulin precursor analogs of the invention during fermentation, protecting the N-terminal end of the insulin precursor or insulin precursor analog against the proteolytic activity of yeast proteases such as DPAP. The presence of an N-terminal extension on the insulin precursor or insulin precursor analog may also serve as a protection of the N-terminal amino group during chemical processing of the protein, i.e. it may serve as a substitute for a BOC (t-butyl-oxycarbonyl) or similar protecting group.

The N-terminal extension may be removed from the recovered insulin precursor or insulin precursor analog by means of a proteolytic enzyme which is specific for a basic amino acid (e.g., Lys) so that the terminal extension is cleaved off at the Lys residue. Examples of such proteolytic enzymes are trypsin or *Achromobacter lyticus* protease.

After secretion to the culture medium and recovery, the insulin precursor or insulin precursor analogs of the invention will be subjected to various in vitro procedures to remove the possible N-terminal extension sequence and the mini C-peptide to give insulin or the desired insulin analog. Such methods include enzymatic conversion by means of trypsin or an *Achromobacter lyticus* protease in the presence of an L-threonine ester followed by conversion of the threonine ester of the insulin or insulin analog into insulin or the insulin analog by basic or acid hydrolysis as described in US patent specification No. 4,343,898 or 4,916,212 or Research Disclosure, September 1994/487 the disclosures of which are incorporated by reference hereinto.

As described below, I Ps with synthetic C-peptides were constructed featuring a Gly residue (Examples 1). A *Saccharomyces cerevisiae* expression plasmid containing a DNA sequences of formula I was constructed by PCR and used to transform a *S. cerevisiae* host cell. The amount of insulin analog produced was measured as a percentage of the control level Asp^{B28}IP lacking mini C-peptide (Table 1). The novel C-peptides of the invention containing a Gly in the sequence X₁ of the mini C-peptide increased yields by up to 4-fold levels.

As described below in Example 2 for Asp^{B28}IP(Asp Gly Lys), the mini C-peptides of the invention result in a region of flexibility between B27 and A1 which all allow a proximity of A1 to B27 measured as the atomic distance between A1 (atom CA) and B27 (atom CG2) (e.g., less than 5 Å). Accordingly, the invention encompasses mini C-peptide constructs which induce the structural effects shown in Example 2 below.

The present invention is described in further detail in the following examples which are not in any way intended to limit the scope of the invention as claimed. The attached Figures are meant to be considered as integral parts of the specification and description of the invention.

### EXAMPLES

### General Procedures

All expressions plasmids are of the C-POT type, similar to those described in EP 171 142, which are characterized by containing the *Schizosaccharomyces pombe* triose phosphate isomerase gene (POT) for the purpose of plasmid selection and stabilization in *S. cerevisiae.* The plasmids furthermore contain the *S*. *cerevisiae* triose phosphate isomerase promoter and terminator. These sequences are similar to the corresponding sequences in plasmid pKFN1003 (described in WO 90/100075) as are all sequences except the sequence of the *Eco*RI*-Xba*I fragment encoding the fusion of the leader and insulin precursor. In order to express different fusion proteins, the *Eco*RI-*Xba*I fragment of pKFN1003 is simply replaced by an *Eco*RI*-Xba*I fragment encoding the leader insulin precursor or leader insulin precursor analog of interest. Such *Eco*RI-*Xba*I fragments may be synthesized using synthetic oligonucleotides and PCR according to standard techniques.

Yeast transformants were prepared by transformation of the host strain: *S*. *cerevisiae* strain MT663 (*MATa*/*MATα pep4-3*/*pep4-3 HIS4*/*his4 tpi::LEU2*/*tpi::LEU2* Cir⁺). The yeast strain MT663 was deposited in the Deutsche Sammlung von Mikroorganismen und Zellkulturen in connection with filing WO 92/11378 and was given the deposit number DSM 6278.

MT663 was grown on YPGaL (1% Bacto yeast extract, 2% Bacto peptone, 2% galactose, 1% lactate) to an O.D. at 600 nm of 0.6. 100 ml of culture was harvested by centrifugation, washed with 10 ml of water, recentrifuged and resuspended in 10 ml of a solution containing 1.2 M sorbitol, 25 mM Na₂EDTA pH = 8.0 and 6.7 mg/ml dithiotreitol. The suspension was incubated at 30°C for 15 minutes, centrifuged and the cells resuspended in 10 ml of a solution containing 1.2 M sorbitol, 10 mM Na₂EDTA, 0.1 M sodium citrate, pH 0 5.8, and 2 mg Novozym®234. The suspension was incubated at 30°C for 30 minutes, the cells collected by centrifugation, washed in 10 ml of 1.2 M sorbitol and 10 ml of CAS (1.2 M sorbitol, 10 mM CaCl₂, 10 mM Tris HCl (Tris = Tris(hydroxymethyl)aminomethane) pH = 7.5) and resuspended in 2 ml of CAS. For transformation, 1 ml of CAS-suspended cells was mixed with approx. 0.1 mg of plasmid DNA and left at room temperature for 15 minutes. 1 ml of (20% polyethylene glycol 4000, 10 mM CaCl₂, 10 mM Tris HCl, pH = 7.5) was added and the mixture left for a further 30 minutes at room temperature. The mixture was centrifuged and the pellet resuspended in 0.1 ml of SOS (1.2 M sorbitol, 33% v/v YPD, 6.7 mM CaCl₂) and incubated at 30°C for 2 hours. The suspension was then centrifuged and the pellet resuspended in 0.5 ml of 1.2 M sorbitol. Then, 6 ml of top agar (the SC medium of Sherman et al. (1982) Methods in Yeast Genetics, Cold Spring Harbor Laboratory) containing 1.2 M sorbitol plus 2.5% agar) at 52°C was added and the suspension poured on top of plates containing the same agar-solidified, sorbitol containing medium.

*S. cerevisiae* stain MT663 transformed with expression plasmids were grown in YPD for 72 h at 30°C. Quantitation of the insulin-precursor yield in the culture supernatants was performed by reverse-phase HPLC analysis with human insulin as an external standard (Snel & Damgaard (1988) Proinsulin heterogenity in pigs. Horm. Metabol. Res. 20:476-488).

### Example 1.

### Construction of Synthetic C-peptides With a Glycine Residue.

Synthetic genes encoding fusion proteins consisting of Asp^{B28}IP associated with a leader sequence consisting of a pre-peptide (signal peptide) and a pro-peptide, were constructed using PCR under standard conditions (Sambrook et al. (1989) Molecular Cloning, Cold Spring Harbor Laboratory Press) and E.H.F. polymerase (Boehringer Mannheim GmbH, Sandhoefer Strasse 116, Mannheim, Germany). The resulting DNA fragments were isolated and digested with endonucleases and purified using the Gene Clean kit (Bio101 Inc., La Jolla, CA, USA). Standard methods were used for DNA ligation and transformation of *E. coli* cells were performed by the CaCl₂ method (Sambrook et al. (1989) *supra*). Plasmids were purified from transformed *E. coli* cells using QIAGEN columns (QIAGEN, Hilden, Germany). Nucleotide sequences were determined using the ALF Pharmacia Biotech DNA sequencing system with purified double-stranded plasmid DNA as template. Oligonucleotide primers for PCR were obtained from DNA technology (Arhus, Denmark).

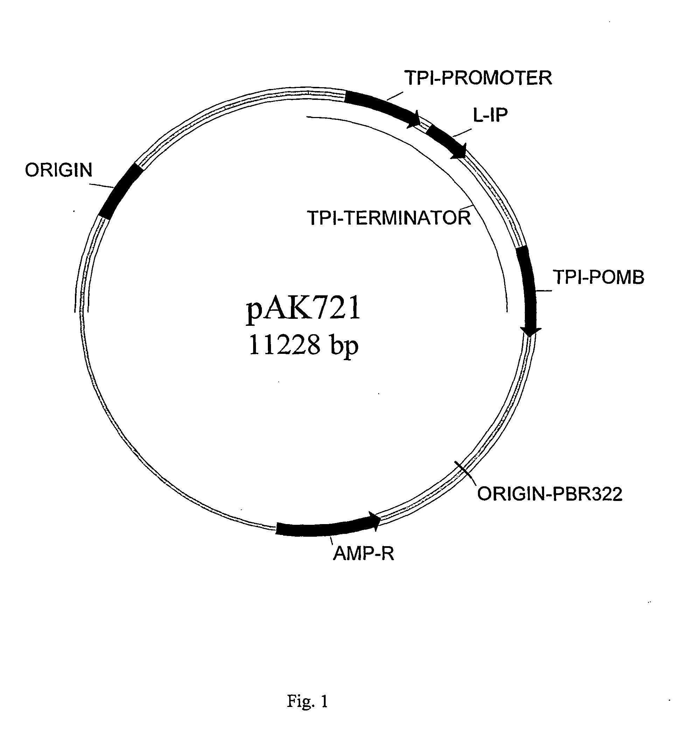

Secretory expression of the Asp^{B28}IP in *S. cerevisiae* was performed using *S. cerevisiae* strain MT663 and the 2 µm based yeast expression vector CPOT (see Fig. 1) as described in Thim, L. et al. (1986) Proc. Natl. Acad. Sci.USA 83:6766-6770. The yeast expression vector contains the *Schizosaccharomyces pombe* triose phosphate isomerase gene (*POT*) for plasmid selection and stabilization in *S. cerevisiae.* Furthermore, the *S. cerevisiae* triose phosphate isomerase gene (*TPI1*) promoter and terminator are used for transcription initiation and termination of the recombinant gene encoding the leader-Asp^{B28}IP fusion protein. Secretion of the Asp^{B28}IP was facilitated by the α-factor leader, although a variety of known yeast leader sequences may be used.

As shown in Fig. 1, the pAK721 *S. cerevisiae* expression plasmid expressing the LA19 leader-EEAEAEAEPK(SEQ ID NO:2)-IP fusion protein was constructed based on the *S. cerevisiae-E. coli* shuttle POT plasmid (U.S. patent 5,871,957). L-IP indicates the fusion protein expression cassette encoding the leader-IP fusion protein, TPI-PROMOTER is the *S. cerevisiae TPI1* promoter and TPI-TERMINATOR is the *S. cerevisiae TPI1* terminator; TPI-POMBE indicates the *S. pombe POT* gene used for selection in *S. cerevisiae;* ORIGIN indicates a *S. cerevisiae* origin of replication derived from the 2µm plasmid; AMP-R indicates the β-lactamase gene conferring resistance toward ampicillin, facilitating selection in *E. coli* and ORIGIN-PBR322 indicates an *E. coli* origin of replication.

DNA encoding a number of fusion proteins of leader sequences and Asp^{B28}IP with different mini-C-peptides was generated by PCR using appropriate oligonucleotides as primers, as described below. Standard methods were used to subclone DNA fragments encoding the leader-Asp^{B28}IP fusion proteins into the CPOT expression vector in the following configuration: leader-Lys-Arg-spacer-Asp^{B28}IP, where Lys Arg is a potential dibasic endoprotease processing site. To optimize processing of the fusion protein by the *S. cerevisiae* Kex2 endoprotease, DNA encoding a spacer peptide, e.g. EEAEAEAPK (SEQ ID NO:3), was inserted between the DNA encoding the leader and the Asp^{B28}IP (Kjeldsen et al. (1996) Gene 170, 107-112.). However, the present of the spacer peptide is not mandatory. The mature Asp^{B28}IP was secreted as a single-chain N-terminally extended insulin precursor analogue with a synthetic mini C-peptide connecting Lys^{B29} and Gly^{A1}. After purification of the Asp^{B28}IP and proteolytic removal of the N-terminal extension and the synthetic mini C-peptide, a threonine amino acid residue (Thr^{B30}) may be added to Lys^{B29} by enzyme-mediated transpeptidation, to generate Asp^{B28} human insulin (Markussen, et al. (1987) in "Peptides 1986" (Theodoropoulos, D., Ed.), pp. 189-194, Walter de Gruyter & Co., Berlin.).

Development of synthetic mini C-peptides was performed by randomization of one or more codon(s) encoding the amino acids in the mini C-peptide. All synthetic mini C-peptides feature an enzymatic processing site (Lys) at the C-terminus which allows enzymatic removal of the synthetic mini C-peptide (U.S. Patent No. 4,916,212, herein specifically incorporated by reference). Randomization was performed using doped oligonucleotides which introduced codon(s) variations at one or more positions of the synthetic mini C-peptides. Typically one of the two primers (oligonucleotides) used for PCR was doped. An example of an oligonucleotides pair used for PCR generation of leader-Asp^{B28}IP with randomized synthetic mini C-peptides used to generated synthetic mini C-peptides with the general formula: Xaa-Gly-Lys (XGK) are as follows:
Primer A: 5'TAAATCTATAACTACAAAAAACACATA-3' (SEQ ID NO:13) and
Primer B: 3'-CCAAAGAAGATGTGACTGTTCNNMCCCTTCCCATAGCAACTTGTTACAACAT-GAAGATAGACAAGAAACATGGTTAACCTTTTGATGACATTGATCAGATCTTTGATTC-5' (SEQ ID NO:14), where N is A, C, G, or T and M is C or A.

PCR was typically performed as indicated below: 5 µl Primer A (20 pmol), 5 µl Primer B (20 pmol), 10 µl 10X PCR buffer, 8 µl dNTP mix, 0.75 µl E.H.F. enzyme, 1 µl pAK1150 plasmid as template (approximately 0.2 µg DNA) (SEQ ID NO:3), and 70.25 µl distilled water.

Typically between 10 and 15 cycles were performed, one cycle typically was 94° C for 45 sec.; 55° C for 1 min; 72° C for 1.5 min. The PCR mixture was subsequently loaded onto an 2 % agarose gel and electrophoresis was performed using standard techniques. The resulting DNA fragment was cut out of the agarose gel and isolated by the Gene Clean kit.

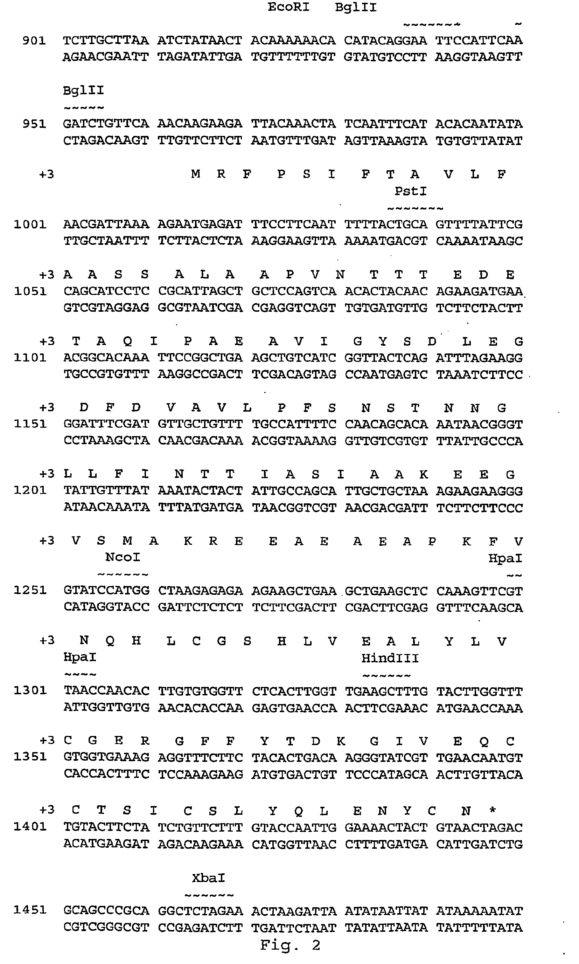

Fig. 2 shows the sequence of pAK1150 DNA used as template for PCR and inferred amino acids of the encoded fusion protein (α-factor-leader-EEAEAEAPK(SEQ ID NO:3)-Asp^{B28}IP of pAK1150 (SEQ ID NO:4 and 5). The pAK1150 plasmid is similar to pAK721 shown in Fig. 1. The α-factor-leader's C-terminus was modified to introduce a Nco I restriction endonuclease site, which changes the inferred amino acid sequences linked to LysArg from SerLeuAsp to SerMetAla. Moreover, the encoded Asp^{B28}IP does not feature a mini C-peptide but Lys^{B29} is directly connected to Gly^{A1}.

The purified PCR DNA fragment was dissolved in water and restriction endonucleases buffer and digested with suitable restriction endonucleases (e.g. Bgl II and Xba I) according to standard techniques. The BglII-XbaI DNA fragments were subjected to agarose electrophoresis and purified using The Gene Clean Kit.

The expression plasmid pAK1150 or a similar plasmid of the CPOT type (see Fig. 1) was digested with the restriction endonucleases Bgl II and Xba I and the vector fragment of 10765 nucleotide basepairs isolated using The Gene Clean Kit.

The two digested and isolated DNA fragments (the vector fragment and the PCR fragment) were ligated together using T4 DNA ligase and standard conditions. The ligation mix was subsequently transformed into a competent E. coli strain (R-, M+) followed by selection with ampicillin resistance. Plasmids from the resulting *E. coli's* were isolated using QIAGEN columns.

The plasmids were subsequently used for transformation of a suitable *S. cerevisiae* strainMT663 (*MATa*/*MATα pep4-3*/*pep4-3 HIS4*/*his4 tpi::LEU2*/*tpi::LEU2* Cir⁺). Individual transformed *S. cerevisiae* clones were grown in liquid culture, and the quantity of Asp^{B28}IP secreted to the culture supernatants were determined by RP-HPLC. The DNA sequence encoding the synthetic mini C-peptide of the expression plasmids from *S. cerevisiae* clones secreting increased quantity of the Asp^{B28}IP were then determined. Subsequently, the identified synthetic mini C-peptide sequence might be subjected to another round of randomization optimization.

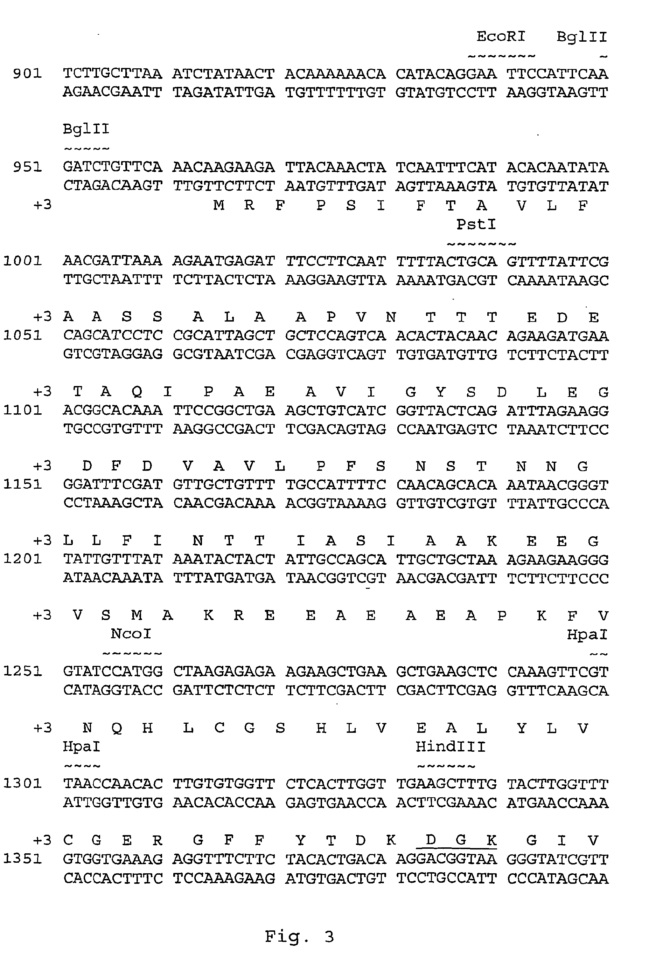

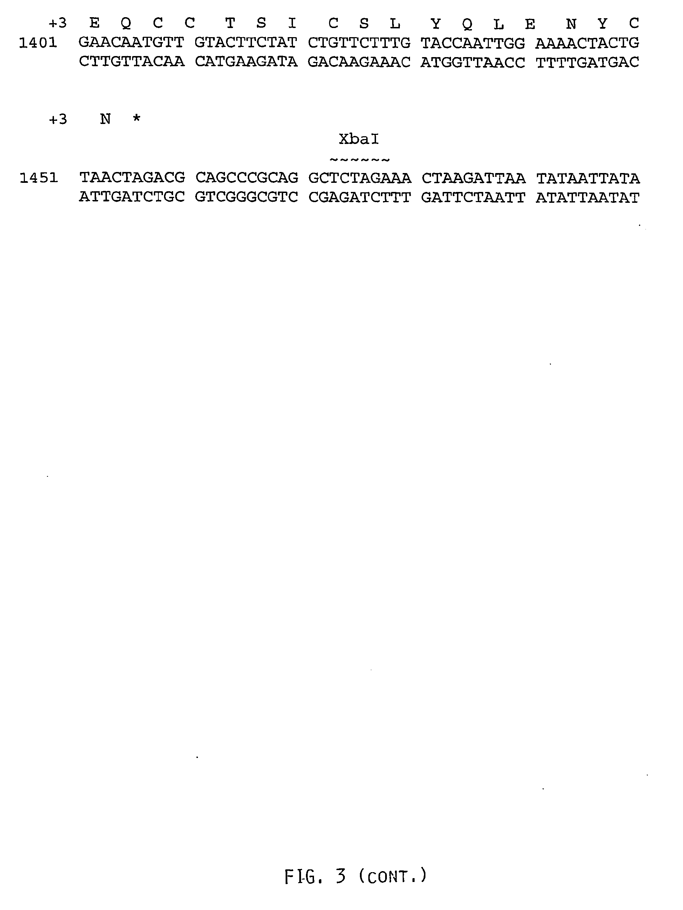

An example on a DNA sequence encoding a leader-Asp^{B28}IP(AspGlyLys) fusion protein featuring a synthetic mini C-peptide (AspGlyLys) resulting from the randomized optimization process described are shown in Fig. 3 (SEQ ID NO:6 and 7).

Table 1 shows the insulin analogue precursors generated by the above method and production yield expressed as a percent of the control. Fermentation was at 30°C for 72 h in 5 ml YPD. Yield of the insulin precursor analogs was determined by RP-HPLC of the culture supernatant, and is expressed relative to the yield of insulin precursor of a control strain.. In the table, "α*" indicates an α-factor leader in which the C-terminus up to the LysArg has been modified from SLD (SerLeuAsp) to SMA (SerMetAla) and "ex4" is an N-terminal extension peptide with the amino acid sequence EEAEAEAPK (SEQ ID NO:3).

**TABLE 1**

| Leader-N-terminal extension | Precursor | C-peptide | Yield* | SEQ ID |
|---|---|---|---|---|
| α*-ex4 | Asp^{B28}IP | - | 100 | |
| α*-ex4 | Asp^{B28}IP | GluGluGlyLys | 245 | SEQ ID NO:1 |
| α*-ex4 | Asp^{B28}IP | GluGlyLys | 350 | |
| α*-ex4 | Asp^{B28}IP | SerGlyLys | 294 | |
| α*-ex4 | Asp^{B28}IP | AsnGlyLys | 341 | |
| α*-ex4 | Asp^{B28}IP | ThrGlyLys | 258 | |
| α*-ex4 | Asp^{B28}IP | AspGlyLys | 428 | |
| α*-ex4 | Asp^{B28}IP | MetGlyLys | 225 | |
| α*-ex4 | Asp^{B28}IP | AlaGlyLys | 243 | |
| α*-ex4 | Asp^{B28}IP | HisGlyLys | 225 | |
| α*-ex4 | Asp^{B28}IP | TyrGlyLys | 214 | |

### Example 2. Structure Determination of Asp^{B28}IP(AspGlyLys) in Aqueous Solution by NMR Spectroscopy.

*NMR spectroscopy.* Samples for NMR were prepared by dissolving the lyophilized protein powder in 10/90 D₂O/H₂O with a 10 mM phosphate buffer and adjusting the pH as desired by addition of small volumes of 1 M DCI or NaOD. All pH meter readings are without correction for isotope effects. Samples of Asp^{B28}IP(AspGlyLys) for NMR were prepared at concentrations ranging from 25µM to 1mM at pH 8.0. Two-dimensional ¹H-¹H NMR spectra of 1mM samples, DQF-COSY (Piantini et al. (1982) J. Am. Chem. Soc. 104:6800-6801, Rance et al. (1983) Biochem. Biophys. Res. Commun. 117:479-485), TOCSY (Braunschweiler et al. (1983) J. Magn. Reson. 53:521-528, Bax et al. (1985) J. Magn. Reson. 65:355-360) and NOESY (Jeener et al. (1979) J. Chem. Phys. 71:4546-4553) were recorded at 600 MHz on a Varian Unity Inova NMR spectrometer equipped with a ¹H/¹³C/¹⁵N triple resonance probe with a self-shielded triple-axis gradient coil using standard pulse sequences from the Varian user library. The operating temperature was set to 27°C. For each phase sensitive two-dimensional NMR spectrum 512 t₁ increments were acquired each with 2048 or 4096 real data points according to the TPPI-States method (Marion et al. (1989) J. Magn. Reson. 85:393-399). Spectral widths of 6983 Hz in both dimensions were used, with the carrier placed exactly on the water resonance which was attenuated by using either saturation between scans for 1.5 seconds or selective excitation by a gradient-tailored excitation pulse sequence (WATERGATE, Piotto et al. (1992) J. Biomol. NMR 2:661-665). DQFCOSY spectra were recorded using a gradient enhanced version applying magic-angle gradients (Mattiello et al. (1996) J. Am. Chem. Soc. 118:3253-3261). For TOCSY spectra mixing times between 30 and 80 ms were used and for NOESY mixing times between 50 and 200 ms.

The processing of the two-dimensional NMR spectra was performed using the software package Xwinnmr (version 2.5, NMR processing software from Bruker Analytische Messtechnik GmbH, D-76275 Ettlingen, Germany). Each dimension was processed with shifted sine-bell apodization and zero-filling performed once in each dimension. Baseline corrections were applied if necessary using Xwinnmr standard procedures. The spectral assignment, cross peak integration, sequence specific assignment, stereo specific assignment, and all other bookkeeping were performed using the program PRONTO (PRONTO Software Development and Distribution, Copenhagen Denmark) (Kjær et al. (1991) NATO ASI Series (Hoch, J. C., Redfield C., & Poulsen, F. M., Eds.) Plenum, New York). Chemical shifts are measured in ppm and the water resonance set to 4.75 ppm.

*Structure calculations.* Distance restraints for the subsequent structure calculation were obtained from integrated NOESY cross peaks classified as either weak, medium or strong corresponding to upper distance restraints of 5.5, 3.3, and 2.7 Å, respectively. For distance restraints involving methyl groups, an additional 0.5 Å was added to the upper limit (Wagner et al. (1985) J. Mol. Biol. 196:611-639). Structure calculations were performed using the hybrid method combining distance geometry (Crippen et al. (1988) Distance Geometry and Molecular Conformation, Research Studies Press, Taunton, Somerset, England; Kuszewski et al. (1992) J.Biomol NMR 2:33-56) and simulated annealing based on the ideas of Nilges et al. (1988) FEBS Lett. 229:317-324 using X-PLOR 3.0 (Brünger (1992) X-PLOR Version 3.1: A System for X-ray Crystallography and NMR, Yale University Press, New Haven) according to the examples given by the X-PLOR manual (dg_sub_embed.inp, dgsa.inp, refine.inp). Residue numbers are derived from standard insulin residue numbering, residues in the B-chain are numbered B1-29, residues in the C-peptide (e. g. AspGlyLys) are numbered C1-C3 and residues in the A-chain are numbered A1-A21.

Spectral assignment of the NMR spectra followed for most resonances the standard sequential assignment procedure described by Wüthrich (1986 NMR of Proteins and Nucleic Acids, Wiley, New York). The standard assignment procedure fails when the amid proton of a particular amino acid residue exchanges to rapidly with protons in the water. At pH 8.0 this occurs for several amino acid residues, however, comparison with earlier mutant insulin NMR spectral assignments and identification of neighboring (in space) amino acid residues through NOEs allow an almost total spectral assignment. Analysis of the NOESY spectra showed that several amino acid residues had a NOE network to the surrounding residues similar to what has previously been determined for other insulin molecules, i.e., human insulin His^{B16} mutant (Ludvigsen et al. (1994) Biochemistry 33:7998-8006) and these similar connections are found for residues B1-B10, B13-B14, B17-B24 and A4-A21. Additionally the dihedral angle restraints for the above listed residues were adopted from those used previously (Ludvigsen et al. (1994) *supra*).

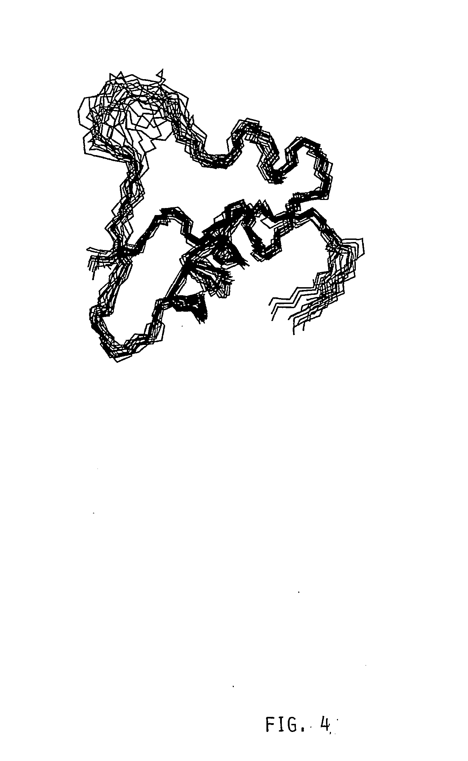

Several amino acids in particular B27-B29, C1-C3, A1-A3 have cross peaks patterns which are consistent with peptide chains that are less well ordered than commonly well-defined secondary structural elements. Thus additional NOEs were converted into distance restraints without any further classification than upper limits of 5.5 Å or 6.0 Å if a methyl group were included. An ensemble of 20 converged structures (Fig. 4) was calculated and the relevant parameters listed in Table 2 for the converged structures. Each NOE here identical to a distance restraint is only counted once even though it might occur several times in the NOESY spectrum. Ramachandran plot quality assessment is standard quality parameters to evaluate local geometry quality. In general the described quality parameters are comparable to 2.5 Å resolution of X-ray based protein structures (Laskowski et al. (1996) J. Biomol. NMR 8:477-486).

**TABLE 2**

| Structural quality assessment | | | Asp^{B28}IP(AspGlyLys) |
|---|---|---|---|
| Number of NOEs | Total | | 913 |
| | Intra | | 454 |
| | short range (within 5 residue positions away but not intra NOEs) | | 297 |
| | Long range (more than 5 residue positions away) | | 162 |
| Violations of NOEs >0.4 Å (average for 20 structures) | | | 0 |
| RMS of NOE violations | | | 0.020(± 0.002) Å |
| RMS of dihedral angle restraints | | | 0.32(±0.12)° |
| Deviations from ideal geometry | | Impropers | 0.37(± 0.05)° |
| | | Angles | 0.43(±0.03) ° |
| | | Bonds | 0.0034(±0.0002) Å |
| Ramachandran Plot (Procheck, Laskowski *et al,* 1996) | Favoured regions | | 76.1 % |
| | additional allowed regions | | 20.8 % |
| | generously allowed regions | | 2.2 % |
| | disallowed regions | | 1.0 % |

### Description of the calculated structure.

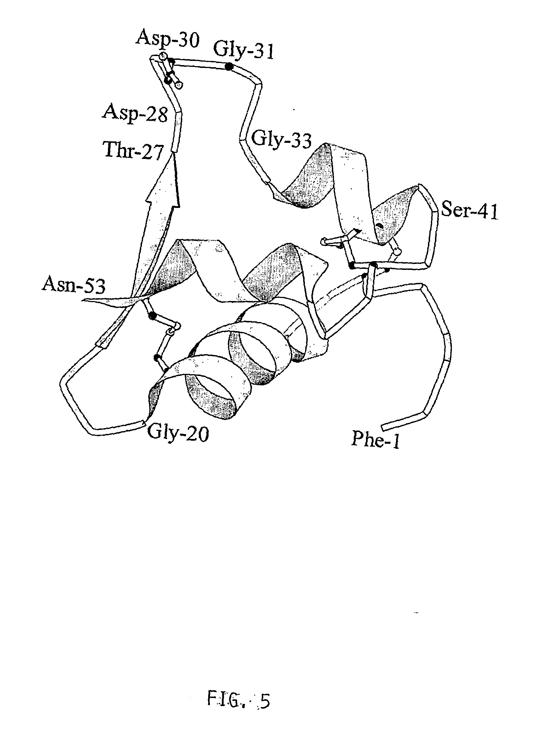

A representative structure resembling the average of the ensemble is displayed in Fig 5. Asp^{B28}IP(AspGlyLys) is structurally similar to the native insulin structure for regions comprising residues B1-B10, B14-B23, A4-A21. The differences are mostly pronounced for regions in the vicinity of the connecting peptide in positions B26-B29, C1-C3, A1-A3 and less pronounced for residues B11-B13. The structure of Asp^{B28}IP(AspGlyLys) near the C-peptide is strikingly different from the native like structure in solution (Ludvigsen (1994) *supra*) and Asp^{B28}IP(AlaAlaLys) structure in the crystal phase (Whittingham et al. (1998) Biochemistry 37:11516-11523). The connecting peptide of Asp^{B28}IP(AspGlyLys) is poorly determined in terms of accuracy, but a few structural restraints obtained from the NOESY spectra (NOEs between Thr^{B27} and Gly^{C2} and between Thr^{B27} and Gly^{A1}) clearly indicate important structural arrangements of the C-peptide. The relative intense NOEs between Thr^{B27} (methyl group HG2) and Gly^{C2} (atom HA) and between Thr^{B27} (methyl group HG2) and Gly^{A1} (atom HA) in a flexible region shows that these proton pairs are close in space (<5 A). The tight arrangement of Thr^{B27}, Gly² and Gly^{A1} defined as the atomic distance between B27 (CG2) and A1 (CA) is less than 5Å, not seen previously in any single chain insulin molecule shows that the C-peptide accommodates this structural arrangement and in fact the C-peptide can do this in several ways which appears to be a prerequisite for the C-peptide. However, it is clear that the presence of Glycine in the connecting peptide allows more flexibility in the connecting peptide and subsequently less structural constraints are imposed on the neighboring amino acids in their quest to accommodate an optimal packing with the remainder of the insulin molecule. Secondly the arrangement of Asp^{B28}, Lys^{B29}, Asp^{C1} and Lys^{C3} charged side-chains creates a highly polar surface compared to other connecting peptides.

Under the conditions used for NMR both the spectra Asp^{B28}IP(AspGlyLys) are influenced by some degree of self-association but the exchange between monomer and dimer is on the timescale of NMR only observed here as an average between the two states. Below concentrations of 0.2 mM the degree of self-association does not change as seen by NMR at even lower concentrations (at least until 25 µM). Table 3 shows chemical shifts of Asp^{B28}IP(AspGlyLys) at 27° Celcius obtained at 600 MHz, pH 8 in 10%/90% D₂O/H₂O with 10 mM phosphate buffer. Chemical shifts are referenced by setting the residual water signal to 4.75 ppm. N/A means no assignment. Asp^{B28}IP(AspGlyLys) assignments (1-29 = B1-B29; 30-32 = C1-C3 and 33-53 =A1-A21). Table 4 provides the atomic coordinates of Asp^{B28}IP(AspGlyLys) in PDB format. The structure selected to represent the ensemble (Fig. 5 and Table 4 atomic coordinates) has 84.8% residues in "favored" regions and 15.2% in "additionally allowed" regions of the ramachandran plot as described in Table 2.

**Table 3 NMR spectral assignments for Asp^{B28}IP(AspGlyLys)**

| Spin system | HN | HA | Other: |
|---|---|---|---|
| Phe-1 | | 4.52 | HB#a: N/A, HB#b: 2.992, HD#: 7.087, HE#: 7.203, HZ: 7.145 |
| Val-2 | 7.70 | 3.99 | HB: 1.912, HG#a: 0.797, HG#b: N/A |
| Asn-3 | | 4.50 | HB#a: 2.989, HB#b: 2.321, HD2#a: 7.372, HD2#b: 6.920 |
| Glu-4 | | 4.38 | HB#a: 1.999, HB#b: 2.103 |
| His-5 | | 4.31 | HB#a: 3.314, HB#b: 2.978, HD2: 6.798, HE1: 7.595 |
| Leu-6 | | 4.44 | HB#a: 1.648, HB#b: N/A, HG: 1.503, HD#a: 0.755, HD#b: 0.662 |
| Cys-7 | 8.28 | 4.87 | HB#a: 2.925, HH#b: 3.143 |
| Gly-8 | | 3.94, 3.76 | |
| Ser-9 | | 4.05 | HB#: 3.812 |
| His-10 | 8.55 | 4.38 | HB#a: 3.124, HB#b: 3.282, HD2: 7.081, HE1: 7.708 |
| Leu-11 | 6.93 | 3.85 | HB#a: 1.749, HB#b: 1.175, HG: 1.222, HD#a: 0.602, HD#b: 0.722 |
| Val-12 | 7.02 | 3.17 | HB: 2.031, HG#a: 0.970, HG#b: N/A |
| Glu-13 | 7.84 | 3.97 | HB#a: 2.025, HG#a: 2.222, HG#b: 2.383 |
| Ala-14 | 7.55 | 3.94 | HB#: 1.253 |
| Leu-15 | 7.91 | 3.59 | HB#a: 0.905, HH#b: 0.067, HG: 1.075, HD#a: 0.377, HD#b: -0.051 |
| Tyr-16 | 8.10 | 4.30 | HB#a: 3.064, HD#: 7.193, HE#: 6.771 |
| Leu-17 | 7.66 | 4.02 | HB#a: N/A, HH#b: 1.844, HG: 1.712, HD#a: 0.892, HD#b: 0.872 |
| Val-18 | 8.27 | 3.68 | HB: 1.882, HG#a: 0.945, HG#b: 0.792 |
| Cys-19 | 8.69 | 4.76 | HB#a: 2.759, HB#b: 3.254 |
| Gly-20 | 7.78 | 3.90, 3.76 | |
| Arg-22 | 8.03 | 4.08 | HB#a: 1.801, HH#b: 1.845, HG#a: 2.041, HG#b: 2.088, HD#a: 3.352, HD#b: 3.280 |
| Gly-23 | 7.23 | 4.07, 3.67 | |
| Phe-24 | 7.37 | 5.37 | HB#a: 2.857, HB#b: 3.013, HD#: 6.502, HE#: 6.688, HZ: 6.944 |
| Phe-25 | 8.51 | 4.87 | HB#a: 3.151, HB#b: 3.321, HD#: 7.162, HE#: 7.073 |
| Tyr-26 | 8.17 | 4.69 | HB#a: 2.920, HH#b: 3.168, HD#: 7.029, HE#: 6.662 |
| Thr-27 | 7.80 | 5.06 | HB: 3.965, HG2#: 1.198 |
| Asp-28 | 8.43 | 4.50 | HH#a: 2.764, HB#b: 2.660 |
| Lys-29 | | | |
| Asp-30 | 8.06 | 4.76 | HB#a: 2.615, HB#b: 2.821 |
| Gly-31 | 8.15 | 4.14, 3.58 | |
| Lys-32 | | | |
| Gly-33 | | 4.03, 4.87 | |
| Ile-34 | 8.16 | 3.78 | HB: N/A, HG1#a: 0.730, HG1#b: 0.885, HG2#: 0.722, HD#: 0.345 |
| Val-35 | 8.08 | 3.54 | HB: 1.966, HB: N/A, HG#a: 0.862, HG#b: 0.957 |
| Glu-36 | 8.23 | 4.11 | HB#a: 2.094, HG#a: 2.249 |
| Gln-37 | 7.79 | 4.09 | HB#: N/A, HG#: N/A |
| Cys-38 | 8.11 | 5.04 | HB#a: 3.273, HB#b: 2.695 |
| Cys-39 | 8.33 | 4.86 | HH#a: 3.732, HB#b: 3.275 |
| Thr-40 | | 4.06 | HB: 4.402, HG2#: 1.188 |
| Ser-41 | 7.23 | 4.62 | HB#a: 3.732, HB#b: 3.874 |
| Ile-42 | 7.74 | 4.18 | HB: 1.488, HG1#a: 1.038, HG2#: 0.604, HD#: 0.446 |
| Cys-43 | 9.61 | 4.95 | HB#a: 3.125 |
| Ser-44 | 8.52 | 4.58 | HB#a: 4.084, HH#b: 3.930 |
| Leu-45 | | 3.88 | HB#a: N/A, HB#b: 1.452, HG: 1.543, HD#a: 0.812, HD#b: 0.750 |
| Tyr-46 | 7.58 | 4.28 | HB#a: 2.956, HD#: 7.074, HE#: 6.806 |
| Gln-47 | 7.43 | 3.95 | HB#a: 2.287, HB#b: 1.983, HG#a: 2.382, HG#b: 2.140 |
| Leu-48 | 7.70 | 3.99 | HB#a: 1.905, HH#b: 1.329, HG: 1.658, HD#a: 0.666, HD#b: 0.614 |
| Glu-49 | 7.82 | 4.15 | HH#a: N/A, HH#b: 1.946, HG#a: 2.307, HG#b: 2.171 |
| Asn-SO | 7.27 | 4.44 | HB#a: 2.716, HB#b: 2.592 |
| Tyr-51 | 7.88 | 3.94 | HB#a: 3.534, HB#b: 2.573, HD#: 7.192, HE#: 6.682 |
| Cys- 52 | 7.03 | 5.10 | HH#a: 2.711, HB#b: 3.231 |
| Asn-53 | 7.88 | 4.46 | HB#a: 2.475, HH#b: 2.683, HD2#a: 7.480, HD2#b: 6.549 |

**Table 4 Atomic coordinates of Asp²⁸IP(AspGlyLys) in PDB format**

| | | | | | | | | | |
|---|---|---|---|---|---|---|---|---|---|
| ATOM | 1 | CA | PHE | 1 | 5.563 | -10.343 | 1.925 | 1.00 | 0.00 |
| ATOM | 2 | HA | PHE | 1 | 5.191 | -11.286 | 1.550 | 1.00 | 0.00 |
| ATOM | 3 | CB | PHE | 1 | 4.581 | -9.224 | 1.558 | 1.00 | 0.00 |
| ATOM | 4 | HB1 | PHE | 1 | 5.131 | -8.325 | 1.323 | 1.00 | 0.00 |
| ATOM | 5 | HB2 | PHE | 1 | 3.923 | -9.037 | 2.393 | 1.00 | 0.00 |
| ATOM | 6 | CG | PHE | 1 | 3.765 | -9.641 | 0.357 | 1.00 | 0.00 |
| ATOM | 7 | CD1 | PHE | 1 | 3.789 | -8.866 | -0.809 | 1.00 | 0.00 |
| ATOM | 8 | HD1 | PHE | 1 | 4.392 | -7.970 | -0.851 | 1.00 | 0.00 |
| ATOM | 9 | CD2 | PHE | 1 | 2.985 | -10.800 | 0.410 | 1.00 | 0.00 |
| ATOM | 10 | HD2 | PHE | 1 | 2.968 | -11.397 | 1.311 | 1.00 | 0.00 |
| ATOM | 11 | CE1 | PHE | 1 | 3.033 | -9.253 | -1.921 | 1.00 | 0.00 |
| ATOM | 12 | HE1 | PHE | 1 | 3.052 | -8.657 | -2.822 | 1.00 | 0.00 |
| ATOM | 13 | CE2 | PHE | 1 | 2.229 | -11.187 | -0.701 | 1.00 | 0.00 |
| ATOM | 14 | HE2 | PHE | 1 | 1.627 | -12.083 | -0.658 | 1.00 | 0.00 |
| ATOM | 15 | CZ | PHE | 1 | 2.252 | -10.414 | -1.867 | 1.00 | 0.00 |
| ATOM | 16 | HZ | PHE | 1 | 1.670 | -10.713 | -2.726 | 1.00 | 0.00 |
| ATOM | 17 | C | PHE | 1 | 6.925 | -10.049 | 1.295 | 1.00 | 0.00 |
| ATOM | 18 | O | PHE | 1 | 7.945 | -10.088 | 1.957 | 1.00 | 0.00 |
| ATOM | 19 | N | PHE | 1 | 5.702 | -10.416 | 3.406 | 1.00 | 0.00 |
| ATOM | 20 | HT1 | PHE | 1 | 6.005 | -9.491 | 3.772 | 1.00 | 0.00 |
| ATOM | 21 | HT2 | PHE | 1 | 6.412 | -11.136 | 3.653 | 1.00 | 0.00 |
| ATOM | 22 | HT3 | PHE | 1 | 4.787 | -10.672 | 3.829 | 1.00 | 0.00 |
| ATOM | 23 | N | VAL | 2 | 6.943 | -9.757 | 0.020 | 1.00 | 0.00 |
| ATOM | 24 | HN | VAL | 2 | 6.105 | -9.735 | -0.486 | 1.00 | 0.00 |
| ATOM | 25 | CA | VAL | 2 | 8.231 | -9.458 | -0.667 | 1.00 | 0.00 |
| ATOM | 26 | HA | VAL | 2 | 9.024 | -10.032 | -0.210 | 1.00 | 0.00 |
| ATOM | 27 | CB | VAL | 2 | 8.122 | -9.830 | -2.148 | 1.00 | 0.00 |
| ATOM | 28 | HB | VAL | 2 | 7.420 | -9.167 | -2.633 | 1.00 | 0.00 |
| ATOM | 29 | CG1 | VAL | 2 | 9.494 | -9.691 | -2.810 | 1.00 | 0.00 |
| ATOM | 30 | HG11 | VAL | 2 | 9.807 | -8.658 | -2.773 | 1.00 | 0.00 |
| ATOM | 31 | HG12 | VAL | 2 | 9.431 | -10.012 | -3.840 | 1.00 | 0.00 |
| ATOM | 32 | HG13 | VAL | 2 | 10.211 | -10.304 | -2.285 | 1.00 | 0.00 |
| ATOM | 33 | CG2 | VAL | 2 | 7.638 | -11.275 | -2.277 | 1.00 | 0.00 |
| ATOM | 34 | HG21 | VAL | 2 | 6.763 | -11.417 | -1.660 | 1.00 | 0.00 |
| ATOM | 35 | HG22 | VAL | 2 | 8.418 | -11.948 | -1.954 | 1.00 | 0.00 |
| ATOM | 36 | HG23 | VAL | 2 | 7.389 | -11.481 | -3.308 | 1.00 | 0.00 |
| ATOM | 37 | C | VAL | 2 | 8.542 | -7.967 | -0.542 | 1.00 | 0.00 |
| ATOM | 38 | O | VAL | 2 | 7.869 | -7.135 | -1.120 | 1.00 | 0.00 |
| ATOM | 39 | N | ASN | 3 | 9.562 | -7.624 | 0.206 | 1.00 | 0.00 |
| ATOM | 40 | HN | ASN | 3 | 10.089 | -8.316 | 0.658 | 1.00 | 0.00 |
| ATOM | 41 | CA | ASN | 3 | 9.926 | -6.186 | 0.370 | 1.00 | 0.00 |
| ATOM | 42 | HA | ASN | 3 | 9.083 | -5.644 | 0.771 | 1.00 | 0.00 |
| ATOM | 43 | CB | ASN | 3 | 11.112 | -6.067 | 1.329 | 1.00 | 0.00 |
| ATOM | 44 | HB1 | ASN | 3 | 11.492 | -5.057 | 1.310 | 1.00 | 0.00 |
| ATOM | 45 | HB2 | ASN | 3 | 11.892 | -6.752 | 1.025 | 1.00 | 0.00 |
| ATOM | 46 | CG | ASN | 3 | 10.654 | -6.411 | 2.748 | 1.00 | 0.00 |
| ATOM | 47 | OD1 | ASN | 3 | 9.911 | -7.350 | 2.949 | 1.00 | 0.00 |
| ATOM | 48 | ND2 | ASN | 3 | 11.070 | -5.683 | 3.748 | 1.00 | 0.00 |
| ATOM | 49 | HD21 | ASN | 3 | 11.669 | -4.925 | 3.586 | 1.00 | 0.00 |
| ATOM | 50 | HD22 | ASN | 3 | 10.781 | -5.894 | 4.660 | 1.00 | 0.00 |
| ATOM | 51 | C | ASN | 3 | 10.308 | -5.602 | -0.991 | 1.00 | 0.00 |
| ATOM | 52 | O | ASN | 3 | 11.414 | -5.782 | -1.466 | 1.00 | 0.00 |
| ATOM | 53 | N | GLN | 4 | 9.395 | -4.909 | -1.623 | 1.00 | 0.00 |
| ATOM | 54 | HN | GLN | 4 | 8.512 | -4.784 | -1.217 | 1.00 | 0.00 |
| ATOM | 55 | CA | GLN | 4 | 9.688 | -4.313 | -2.957 | 1.00 | 0.00 |
| ATOM | 56 | HA | GLN | 4 | 10.705 | -3.950 | -2.973 | 1.00 | 0.00 |
| ATOM | 57 | CB | GLN | 4 | 9.509 | -5.381 | -4.038 | 1.00 | 0.00 |
| ATOM | 58 | HB1 | GLN | 4 | 8.468 | -5.655 | -4.103 | 1.00 | 0.00 |
| ATOM | 59 | HB2 | GLN | 4 | 10.096 | -6.249 | -3.779 | 1.00 | 0.00 |
| ATOM | 60 | CG | GLN | 4 | 9.977 | -4.834 | -5.389 | 1.00 | 0.00 |
| ATOM | 61 | HG1 | GLN | 4 | 10.940 | -4.358 | -5.272 | 1.00 | 0.00 |
| ATOM | 62 | HG2 | GLN | 4 | 9.260 | -4.114 | -5.754 | 1.00 | 0.00 |
| ATOM | 63 | CD | GLN | 4 | 10.101 | -5.986 | -6.387 | 1.00 | 0.00 |
| ATOM | 64 | OE1 | GLN | 4 | 9.108 | -6.515 | -6.849 | 1.00 | 0.00 |
| ATOM | 65 | NE2 | GLN | 4 | 11.285 | -6.401 | -6.744 | 1.00 | 0.00 |
| ATOM | 66 | HE21 | GLN | 4 | 12.086 | -5.975 | -6.373 | 1.00 | 0.00 |
| ATOM | 67 | HE22 | GLN | 4 | 11.374 | -7.140 | -7.381 | 1.00 | 0.00 |
| ATOM | 68 | C | GLN | 4 | 8.728 | -3.150 | -3.217 | 1.00 | 0.00 |
| ATOM | 69 | O | GLN | 4 | 7.710 | -3.016 | -2.564 | 1.00 | 0.00 |
| ATOM | 70 | N | HIS | 5 | 9.053 | -2.304 | -4.160 | 1.00 | 0.00 |
| ATOM | 71 | HN | HIS | 5 | 9.883 | -2.434 | -4.665 | 1.00 | 0.00 |
| ATOM | 72 | CA | HIS | 5 | 8.175 | -1.136 | -4.467 | 1.00 | 0.00 |
| ATOM | 73 | HA | HIS | 5 | 7.882 | -0.660 | -3.543 | 1.00 | 0.00 |
| ATOM | 74 | CB | HIS | 5 | 8.952 | -0.133 | -5.327 | 1.00 | 0.00 |
| ATOM | 75 | HB1 | HIS | 5 | 8.355 | 0.752 | -5.481 | 1.00 | 0.00 |
| ATOM | 76 | HB2 | HIS | 5 | 9.190 | -0.582 | -6.281 | 1.00 | 0.00 |
| ATOM | 77 | CG | HIS | 5 | 10.221 | 0.235 | -4.607 | 1.00 | 0.00 |
| ATOM | 78 | ND1 | HIS | 5 | 10.323 | 1.365 | -3.815 | 1.00 | 0.00 |
| ATOM | 79 | HD1 | HIS | 5 | 9.636 | 2.051 | -3.698 | 1.00 | 0.00 |
| ATOM | 80 | CD2 | HIS | 5 | 11.431 | -0.401 | -4.505 | 1.00 | 0.00 |
| ATOM | 81 | HD2 | HIS | 5 | 11.691 | -1.314 | -5.005 | 1.00 | 0.00 |
| ATOM | 82 | CE1 | HIS | 5 | 11.553 | 1.366 | -3.267 | 1.00 | 0.00 |
| ATOM | 83 | HE1 | HIS | 5 | 11.906 | 2.105 | -2.570 | 1.00 | 0.00 |
| ATOM | 84 | NE2 | HIS | 5 | 12.270 | 0.312 | -3.659 | 1.00 | 0.00 |
| ATOM | 85 | C | HIS | 5 | 6.922 | -1.614 | -5.206 | 1.00 | 0.00 |
| ATOM | 86 | O | HIS | 5 | 6.951 | -2.590 | -5.931 | 1.00 | 0.00 |
| ATOM | 87 | N | LEU | 6 | 5.819 | -0.941 | -5.005 | 1.00 | 0.00 |
| ATOM | 88 | HN | LEU | 6 | 5.824 | -0.170 | -4.403 | 1.00 | 0.00 |
| ATOM | 89 | CA | LEU | 6 | 4.546 | -1.353 | -5.667 | 1.00 | 0.00 |
| ATOM | 90 | HA | LEU | 6 | 4.633 | -2.370 | -6.017 | 1.00 | 0.00 |
| ATOM | 91 | CB | LEU | 6 | 3.398 | -1.259 | -4.658 | 1.00 | 0.00 |
| ATOM | 92 | HB1 | LEU | 6 | 2.510 | -1.694 | -5.086 | 1.00 | 0.00 |
| ATOM | 93 | HB2 | LEU | 6 | 3.213 | -0.219 | -4.425 | 1.00 | 0.00 |
| ATOM | 94 | CG | LEU | 6 | 3.766 | -2.012 | -3.378 | 1.00 | 0.00 |
| ATOM | 95 | HG | LEU | 6 | 4.726 | -1.669 | -3.022 | 1.00 | 0.00 |
| ATOM | 96 | CD1 | LEU | 6 | 2.702 | -1.755 | -2.306 | 1.00 | 0.00 |
| ATOM | 97 | HD11 | LEU | 6 | 2.518 | -2.664 | -1.752 | 1.00 | 0.00 |
| ATOM | 98 | HD12 | LEU | 6 | 1.786 | -1.429 | -2.775 | 1.00 | 0.00 |
| ATOM | 99 | HD13 | LEU | 6 | 3.052 | -0.989 | -1.629 | 1.00 | 0.00 |
| ATOM | 100 | CD2 | LEU | 6 | 3.837 | -3.510 | -3.681 | 1.00 | 0.00 |
| ATOM | 101 | HD21 | LEU | 6 | 4.620 | -3.694 | -4.401 | 1.00 | 0.00 |
| ATOM | 102 | HD22 | LEU | 6 | 2.891 | -3.840 | -4.083 | 1.00 | 0.00 |
| ATOM | 103 | HD23 | LEU | 6 | 4.050 | -4.052 | -2.771 | 1.00 | 0.00 |
| ATOM | 104 | C | LEU | 6 | 4.239 | -0.428 | -6.848 | 1.00 | 0.00 |
| ATOM | 105 | O | LEU | 6 | 3.838 | 0.706 | -6.667 | 1.00 | 0.00 |
| ATOM | 106 | N | CYS | 7 | 4.408 | -0.908 | -8.052 | 1.00 | 0.00 |
| ATOM | 107 | HN | CYS | 7 | 4.721 | -1.830 | -8.171 | 1.00 | 0.00 |
| ATOM | 108 | CA | CYS | 7 | 4.113 | -0.063 | -9.246 | 1.00 | 0.00 |
| ATOM | 109 | HA | CYS | 7 | 3.509 | 0.781 | -8.947 | 1.00 | 0.00 |
| ATOM | 110 | HB1 | CYS | 7 | 5.206 | 1.052 | -10.719 | 1.00 | 0.00 |
| ATOM | 111 | HB2 | CYS | 7 | 6.026 | -0.406 | -10.157 | 1.00 | 0.00 |
| ATOM | 112 | C | CYS | 7 | 3.348 | -0.893 | -10.279 | 1.00 | 0.00 |
| ATOM | 113 | O | CYS | 7 | 3.726 | -2.004 | -10.597 | 1.00 | 0.00 |
| ATOM | 114 | CB | CYS | 7 | 5.422 | 0.438 | -9.857 | 1.00 | 0.00 |
| ATOM | 115 | SG | CYS | 7 | 6.321 | 1.417 | -8.626 | 1.00 | 0.00 |
| ATOM | 116 | N | GLY | 8 | 2.272 | -0.359 | -10.798 | 1.00 | 0.00 |
| ATOM | 117 | HN | GLY | 8 | 1.989 | 0.538 | -10.520 | 1.00 | 0.00 |
| ATOM | 118 | CA | GLY | 8 | 1.470 | -1.108 | -11.809 | 1.00 | 0.00 |
| ATOM | 119 | HA1 | GLY | 8 | 2.057 | -1.925 | -12.201 | 1.00 | 0.00 |
| ATOM | 120 | HA2 | GLY | 8 | 1.195 | -0.442 | -12.616 | 1.00 | 0.00 |
| ATOM | 121 | C | GLY | 8 | 0.206 | -1.665 | -11.152 | 1.00 | 0.00 |
| ATOM | 122 | O | GLY | 8 | -0.493 | -0.968 | -10.441 | 1.00 | 0.00 |
| ATOM | 123 | N | SER | 9 | -0.091 | -2.917 | -11.390 | 1.00 | 0.00 |
| ATOM | 124 | HN | SER | 9 | 0.492 | -3.453 | -11.968 | 1.00 | 0.00 |
| ATOM | 125 | CA | SER | 9 | -1.309 | -3.534 | -10.788 | 1.00 | 0.00 |
| ATOM | 126 | HA | SER | 9 | -2.073 | -2.781 | -10.672 | 1.00 | 0.00 |
| ATOM | 127 | CB | SER | 9 | -1.822 | -4.640 | -11.711 | 1.00 | 0.00 |
| ATOM | 128 | HB1 | SER | 9 | -1.181 | -5.508 | -11.621 | 1.00 | 0.00 |
| ATOM | 129 | HB2 | SER | 9 | -1.810 | -4.293 | -12.731 | 1.00 | 0.00 |
| ATOM | 130 | OG | SER | 9 | -3.154 | -4.977 | -11.346 | 1.00 | 0.00 |
| ATOM | 131 | HG | SER | 9 | -3.683 | -5.002 | -12.147 | 1.00 | 0.00 |
| ATOM | 132 | C | SER | 9 | -0.972 | -4.134 | -9.415 | 1.00 | 0.00 |
| ATOM | 133 | O | SER | 9 | -1.845 | -4.340 | -8.594 | 1.00 | 0.00 |
| ATOM | 134 | N | HIS | 10 | 0.284 | -4.419 | -9.164 | 1.00 | 0.00 |
| ATOM | 135 | HN | HIS | 10 | 0.969 | -4.248 | -9.841 | 1.00 | 0.00 |
| ATOM | 136 | CA | HIS | 10 | 0.677 | -5.009 | -7.849 | 1.00 | 0.00 |
| ATOM | 137 | HA | HIS | 10 | 0.159 | -5.946 | -7.710 | 1.00 | 0.00 |
| ATOM | 138 | CB | HIS | 10 | 2.188 | -5.262 | -7.841 | 1.00 | 0.00 |
| ATOM | 139 | HB1 | HIS | 10 | 2.488 | -5.607 | -6.863 | 1.00 | 0.00 |
| ATOM | 140 | HB2 | HIS | 10 | 2.705 | -4.342 | -8.070 | 1.00 | 0.00 |
| ATOM | 141 | CG | HIS | 10 | 2.548 | -6.305 | -8.872 | 1.00 | 0.00 |
| ATOM | 142 | ND1 | HIS | 10 | 1.608 | -6.886 | -9.715 | 1.00 | 0.00 |
| ATOM | 143 | HD1 | HIS | 10 | 0.648 | -6.690 | -9.733 | 1.00 | 0.00 |
| ATOM | 144 | CD2 | HIS | 10 | 3.750 | -6.882 | -9.204 | 1.00 | 0.00 |
| ATOM | 145 | HD2 | HIS | 10 | 4.697 | -6.655 | -8.738 | 1.00 | 0.00 |
| ATOM | 146 | CE1 | HIS | 10 | 2.255 | -7.766 | -10.500 | 1.00 | 0.00 |
| ATOM | 147 | HE1 | HIS | 10 | 1.775 | -8.370 | -11.255 | 1.00 | 0.00 |
| ATOM | 148 | NE2 | HIS | 10 | 3.562 | -7.802 | -10.231 | 1.00 | 0.00 |
| ATOM | 149 | C | HIS | 10 | 0.314 | -4.052 | -6.702 | 1.00 | 0.00 |
| ATOM | 150 | O | HIS | 10 | 0.243 | -4.455 | -5.557 | 1.00 | 0.00 |
| ATOM | 151 | N | LEU | 11 | 0.089 | -2.792 | -6.994 | 1.00 | 0.00 |
| ATOM | 152 | HN | LEU | 11 | 0.156 | -2.485 | -7.921 | 1.00 | 0.00 |
| ATOM | 153 | CA | LEU | 11 | -0.264 | -1.813 | -5.918 | 1.00 | 0.00 |
| ATOM | 154 | HA | LEU | 11 | 0.542 | -1.764 | -5.202 | 1.00 | 0.00 |
| ATOM | 155 | CB | LEU | 11 | -0.474 | -0.420 | -6.548 | 1.00 | 0.00 |
| ATOM | 156 | HB1 | LEU | 11 | -1.303 | -0.471 | -7.239 | 1.00 | 0.00 |
| ATOM | 157 | HB2 | LEU | 11 | 0.417 | -0.143 | -7.090 | 1.00 | 0.00 |
| ATOM | 158 | CG | LEU | 11 | -0.771 | 0.668 | -5.484 | 1.00 | 0.00 |
| ATOM | 159 | HG | LEU | 11 | -0.601 | 1.639 | -5.929 | 1.00 | 0.00 |
| ATOM | 160 | CD1 | LEU | 11 | -2.235 | 0.588 | -5.040 | 1.00 | 0.00 |
| ATOM | 161 | HD11 | LEU | 11 | -2.305 | 0.002 | -4.134 | 1.00 | 0.00 |
| ATOM | 162 | HD12 | LEU | 11 | -2.823 | 0.121 | -5.817 | 1.00 | 0.00 |
| ATOM | 163 | HD13 | LEU | 11 | -2.609 | 1.584 | -4.853 | 1.00 | 0.00 |
| ATOM | 164 | CD2 | LEU | 11 | 0.146 | 0.514 | -4.260 | 1.00 | 0.00 |
| ATOM | 165 | HD21 | LEU | 11 | -0.214 | -0.294 | -3.640 | 1.00 | 0.00 |
| ATOM | 166 | HD22 | LEU | 11 | 0.144 | 1.432 | -3.691 | 1.00 | 0.00 |
| ATOM | 167 | HD23 | LEU | 11 | 1.151 | 0.296 | -4.589 | 1.00 | 0.00 |
| ATOM | 168 | C | LEU | 11 | -1.543 | -2.259 | -5.207 | 1.00 | 0.00 |
| ATOM | 169 | O | LEU | 11 | -1.542 | -2.522 | -4.020 | 1.00 | 0.00 |
| ATOM | 170 | N | VAL | 12 | -2.636 | -2.322 | -5.922 | 1.00 | 0.00 |
| ATOM | 171 | HN | VAL | 12 | -2.608 | -2.089 | -6.874 | 1.00 | 0.00 |
| ATOM | 172 | CA | VAL | 12 | -3.931 | -2.729 | -5.298 | 1.00 | 0.00 |
| ATOM | 173 | HA | VAL | 12 | -4.203 | -1.999 | -4.550 | 1.00 | 0.00 |
| ATOM | 174 | CB | VAL | 12 | -5.020 | -2.770 | -6.379 | 1.00 | 0.00 |
| ATOM | 175 | HB | VAL | 12 | -5.052 | -1.816 | -6.884 | 1.00 | 0.00 |
| ATOM | 176 | CG1 | VAL | 12 | -4.715 | -3.872 | -7.406 | 1.00 | 0.00 |
| ATOM | 177 | HG11 | VAL | 12 | -3.689 | -4.192 | -7.295 | 1.00 | 0.00 |
| ATOM | 178 | HG12 | VAL | 12 | -4.864 | -3.485 | -8.403 | 1.00 | 0.00 |
| ATOM | 179 | HG13 | VAL | 12 | -5.373 | -4.713 | -7.245 | 1.00 | 0.00 |
| ATOM | 180 | CG2 | VAL | 12 | -6.378 | -3.035 | -5.720 | 1.00 | 0.00 |
| ATOM | 181 | HG21 | VAL | 12 | -6.885 | -2.097 | -5.560 | 1.00 | 0.00 |
| ATOM | 182 | HG22 | VAL | 12 | -6.230 | -3.531 | -4.772 | 1.00 | 0.00 |
| ATOM | 183 | HG23 | VAL | 12 | -6.975 | -3.662 | -6.365 | 1.00 | 0.00 |
| ATOM | 184 | C | VAL | 12 | -3.795 | -4.105 | -4.629 | 1.00 | 0.00 |
| ATOM | 185 | O | VAL | 12 | -4.496 | -4.415 | -3.684 | 1.00 | 0.00 |
| ATOM | 186 | N | GLU | 13 | -2.897 | -4.928 | -5.112 | 1.00 | 0.00 |
| ATOM | 187 | HN | GLU | 13 | -2.343 | -4.655 | -5.873 | 1.00 | 0.00 |
| ATOM | 188 | CA | GLU | 13 | -2.716 | -6.281 | -4.505 | 1.00 | 0.00 |
| ATOM | 189 | HA | GLU | 13 | -3.650 | -6.802 | -4.509 | 1.00 | 0.00 |
| ATOM | 190 | CB | GLU | 13 | -1.675 | -7.072 | -5.302 | 1.00 | 0.00 |
| ATOM | 191 | HB1 | GLU | 13 | -1.299 | -7.882 | -4.696 | 1.00 | 0.00 |
| ATOM | 192 | HB2 | GLU | 13 | -0.860 | -6.418 | -5.577 | 1.00 | 0.00 |
| ATOM | 193 | CG | GLU | 13 | -2.319 | -7.642 | -6.567 | 1.00 | 0.00 |
| ATOM | 194 | HG1 | GLU | 13 | -2.926 | -6.883 | -7.038 | 1.00 | 0.00 |
| ATOM | 195 | HG2 | GLU | 13 | -2.939 | -8.488 | -6.306 | 1.00 | 0.00 |
| ATOM | 196 | CD | GLU | 13 | -1.225 | -8.091 | -7.536 | 1.00 | 0.00 |
| ATOM | 197 | OE1 | GLU | 13 | -0.270 | -8.698 | -7.080 | 1.00 | 0.00 |
| ATOM | 198 | OE2 | GLU | 13 | -1.359 | -7.821 | -8.718 | 1.00 | 0.00 |
| ATOM | 199 | C | GLU | 13 | -2.254 | -6.129 | -3.066 | 1.00 | 0.00 |
| ATOM | 200 | O | GLU | 13 | -2.698 | -6.838 | -2.188 | 1.00 | 0.00 |
| ATOM | 201 | N | ALA | 14 | -1.378 | -5.204 | -2.821 | 1.00 | 0.00 |
| ATOM | 202 | HN | ALA | 14 | -1.044 | -4.644 | -3.552 | 1.00 | 0.00 |
| ATOM | 203 | CA | ALA | 14 | -0.885 | -4.986 | -1.439 | 1.00 | 0.00 |
| ATOM | 204 | HA | ALA | 14 | -0.531 | -5.919 | -1.027 | 1.00 | 0.00 |
| ATOM | 205 | CB | ALA | 14 | 0.254 | -3.979 | -1.475 | 1.00 | 0.00 |
| ATOM | 206 | HB1 | ALA | 14 | -0.159 | -2.981 | -1.506 | 1.00 | 0.00 |
| ATOM | 207 | HB2 | ALA | 14 | 0.855 | -4.148 | -2.355 | 1.00 | 0.00 |
| ATOM | 208 | HB3 | ALA | 14 | 0.862 | -4.092 | -0.591 | 1.00 | 0.00 |
| ATOM | 209 | C | ALA | 14 | -2.010 | -4.427 | -0.563 | 1.00 | 0.00 |
| ATOM | 210 | O | ALA | 14 | -1.948 | -4.511 | 0.644 | 1.00 | 0.00 |
| ATOM | 211 | N | LEU | 15 | -3.018 | -3.829 | -1.154 | 1.00 | 0.00 |
| ATOM | 212 | HN | LEU | 15 | -3.038 | -3.744 | -2.129 | 1.00 | 0.00 |
| ATOM | 213 | CA | LEU | 15 | -4.114 | -3.237 | -0.335 | 1.00 | 0.00 |
| ATOM | 214 | HA | LEU | 15 | -3.678 | -2.805 | 0.553 | 1.00 | 0.00 |
| ATOM | 215 | CB | LEU | 15 | -4.815 | -2.131 | -1.125 | 1.00 | 0.00 |
| ATOM | 216 | HB1 | LEU | 15 | -5.783 | -1.935 | -0.691 | 1.00 | 0.00 |
| ATOM | 217 | HB2 | LEU | 15 | -4.936 | -2.445 | -2.153 | 1.00 | 0.00 |
| ATOM | 218 | CG | LEU | 15 | -3.969 | -0.854 | -1.077 | 1.00 | 0.00 |
| ATOM | 219 | HG | LEU | 15 | -2.977 | -1.073 | -1.450 | 1.00 | 0.00 |
| ATOM | 220 | CD1 | LEU | 15 | -4.617 | 0.221 | -1.951 | 1.00 | 0.00 |
| ATOM | 221 | HD11 | LEU | 15 | -5.679 | 0.032 | -2.029 | 1.00 | 0.00 |
| ATOM | 222 | HD12 | LEU | 15 | -4.176 | 0.199 | -2.938 | 1.00 | 0.00 |
| ATOM | 223 | HD13 | LEU | 15 | -4.457 | 1.192 | -1.507 | 1.00 | 0.00 |
| ATOM | 224 | CD2 | LEU | 15 | -3.873 | -0.343 | 0.370 | 1.00 | 0.00 |
| ATOM | 225 | HD21 | LEU | 15 | -3.530 | 0.683 | 0.370 | 1.00 | 0.00 |
| ATOM | 226 | HD22 | LEU | 15 | -3.175 | -0.955 | 0.927 | 1.00 | 0.00 |
| ATOM | 227 | HD23 | LEU | 15 | -4.847 | -0.396 | 0.835 | 1.00 | 0.00 |
| ATOM | 228 | C | LEU | 15 | -5.129 | -4.291 | 0.108 | 1.00 | 0.00 |
| ATOM | 229 | O | LEU | 15 | -5.380 | -4.409 | 1.290 | 1.00 | 0.00 |
| ATOM | 230 | N | TYR | 16 | -5.728 | -5.057 | -0.787 | 1.00 | 0.00 |
| ATOM | 231 | HN | TYR | 16 | -5.524 | -4.964 | -1.743 | 1.00 | 0.00 |
| ATOM | 232 | CA | TYR | 16 | -6.725 | -6.069 | -0.290 | 1.00 | 0.00 |
| ATOM | 233 | HA | TYR | 16 | -7.334 | -5.578 | 0.452 | 1.00 | 0.00 |
| ATOM | 234 | CB | TYR | 16 | -7.655 | -6.628 | -1.371 | 1.00 | 0.00 |
| ATOM | 235 | HB1 | TYR | 16 | -8.424 | -5.905 | -1.580 | 1.00 | 0.00 |
| ATOM | 236 | HB2 | TYR | 16 | -8.115 | -7.527 | -1.002 | 1.00 | 0.00 |
| ATOM | 237 | CG | TYR | 16 | -6.942 | -6.954 | -2.641 | 1.00 | 0.00 |
| ATOM | 238 | CD1 | TYR | 16 | -6.878 | -5.998 | -3.640 | 1.00 | 0.00 |
| ATOM | 239 | HD1 | TYR | 16 | -7.282 | -5.020 | -3.467 | 1.00 | 0.00 |
| ATOM | 240 | CD2 | TYR | 16 | -6.414 | -8.230 | -2.846 | 1.00 | 0.00 |
| ATOM | 241 | HD2 | TYR | 16 | -6.462 | -8.969 | -2.059 | 1.00 | 0.00 |
| ATOM | 242 | CE1 | TYR | 16 | -6.290 | -6.298 | -4.856 | 1.00 | 0.00 |
| ATOM | 243 | HE1 | TYR | 16 | -6.249 | -5.541 | -5.617 | 1.00 | 0.00 |
| ATOM | 244 | CE2 | TYR | 16 | -5.805 | -8.541 | -4.063 | 1.00 | 0.00 |
| ATOM | 245 | HE2 | TYR | 16 | -5.385 | -9.525 | -4.220 | 1.00 | 0.00 |
| ATOM | 246 | CZ | TYR | 16 | -5.748 | -7.574 | -5.078 | 1.00 | 0.00 |
| ATOM | 247 | OH | TYR | 16 | -5.159 | -7.874 | -6.289 | 1.00 | 0.00 |
| ATOM | 248 | HH | TYR | 16 | -5.854 | -7.948 | -6.946 | 1.00 | 0.00 |
| ATOM | 249 | C | TYR | 16 | -5.992 | -7.206 | 0.394 | 1.00 | 0.00 |
| ATOM | 250 | O | TYR | 16 | -6.522 | -7.835 | 1.293 | 1.00 | 0.00 |
| ATOM | 251 | N | LEU | 17 | -4.758 | -7.455 | 0.025 | 1.00 | 0.00 |
| ATOM | 252 | HN | LEU | 17 | -4.336 | -6.915 | -0.674 | 1.00 | 0.00 |
| ATOM | 253 | CA | LEU | 17 | -3.980 | -8.527 | 0.713 | 1.00 | 0.00 |
| ATOM | 254 | HA | LEU | 17 | -4.465 | -9.481 | 0.569 | 1.00 | 0.00 |
| ATOM | 255 | CB | LEU | 17 | -2.558 | -8.573 | 0.151 | 1.00 | 0.00 |
| ATOM | 256 | HB1 | LEU | 17 | -2.022 | -7.685 | 0.448 | 1.00 | 0.00 |
| ATOM | 257 | HB2 | LEU | 17 | -2.602 | -8.628 | -0.925 | 1.00 | 0.00 |
| ATOM | 258 | CG | LEU | 17 | -1.837 | -9.805 | 0.688 | 1.00 | 0.00 |
| ATOM | 259 | HG | LEU | 17 | -1.862 | -9.793 | 1.769 | 1.00 | 0.00 |
| ATOM | 260 | CD1 | LEU | 17 | -2.536 | -11.064 | 0.174 | 1.00 | 0.00 |
| ATOM | 261 | HD11 | LEU | 17 | -1.806 | -11.843 | 0.011 | 1.00 | 0.00 |
| ATOM | 262 | HD12 | LEU | 17 | -3.044 | -10.842 | -0.759 | 1.00 | 0.00 |
| ATOM | 263 | HD13 | LEU | 17 | -3.259 | -11.393 | 0.907 | 1.00 | 0.00 |
| ATOM | 264 | CD2 | LEU | 17 | -0.385 | -9.787 | 0.207 | 1.00 | 0.00 |
| ATOM | 265 | HD21 | LEU | 17 | 0.104 | -8.898 | 0.578 | 1.00 | 0.00 |
| ATOM | 266 | HD22 | LEU | 17 | -0.363 | -9.786 | -0.873 | 1.00 | 0.00 |
| ATOM | 267 | HD23 | LEU | 17 | 0.128 | -10.661 | 0.578 | 1.00 | 0.00 |
| ATOM | 268 | C | LEU | 17 | -3.939 | -8.186 | 2.207 | 1.00 | 0.00 |
| ATOM | 269 | O | LEU | 17 | -3.941 | -9.052 | 3.060 | 1.00 | 0.00 |
| ATOM | 270 | N | VAL | 18 | -3.940 | -6.911 | 2.511 | 1.00 | 0.00 |
| ATOM | 271 | HN | VAL | 18 | -3.959 | -6.242 | 1.798 | 1.00 | 0.00 |
| ATOM | 272 | CA | VAL | 18 | -3.943 | -6.464 | 3.921 | 1.00 | 0.00 |
| ATOM | 273 | HA | VAL | 18 | -3.358 | -7.145 | 4.520 | 1.00 | 0.00 |
| ATOM | 274 | CB | VAL | 18 | -3.370 | -5.042 | 4.015 | 1.00 | 0.00 |
| ATOM | 275 | HB | VAL | 18 | -4.051 | -4.353 | 3.529 | 1.00 | 0.00 |
| ATOM | 276 | CG1 | VAL | 18 | -3.222 | -4.647 | 5.484 | 1.00 | 0.00 |
| ATOM | 277 | HG11 | VAL | 18 | -4.150 | -4.224 | 5.837 | 1.00 | 0.00 |
| ATOM | 278 | HG12 | VAL | 18 | -2.433 | -3.917 | 5.584 | 1.00 | 0.00 |
| ATOM | 279 | HG13 | VAL | 18 | -2.980 | -5.521 | 6.071 | 1.00 | 0.00 |
| ATOM | 280 | CG2 | VAL | 18 | -2.004 | -4.964 | 3.327 | 1.00 | 0.00 |
| ATOM | 281 | HG21 | VAL | 18 | -1.904 | -5.774 | 2.622 | 1.00 | 0.00 |
| ATOM | 282 | HG22 | VAL | 18 | -1.223 | -5.030 | 4.066 | 1.00 | 0.00 |
| ATOM | 283 | HG23 | VAL | 18 | -1.924 | -4.019 | 2.805 | 1.00 | 0.00 |
| ATOM | 284 | C | VAL | 18 | -5.395 | -6.419 | 4.421 | 1.00 | 0.00 |
| ATOM | 285 | O | VAL | 18 | -5.660 | -6.559 | 5.599 | 1.00 | 0.00 |
| ATOM | 286 | N | CYS | 19 | -6.334 | -6.183 | 3.528 | 1.00 | 0.00 |
| ATOM | 287 | HN | CYS | 19 | -6.095 | -6.041 | 2.586 | 1.00 | 0.00 |
| ATOM | 288 | CA | CYS | 19 | -7.765 | -6.079 | 3.939 | 1.00 | 0.00 |
| ATOM | 289 | HA | CYS | 19 | -7.813 | -5.715 | 4.955 | 1.00 | 0.00 |
| ATOM | 290 | HB1 | CYS | 19 | -9.470 | -4.891 | 3.352 | 1.00 | 0.00 |
| ATOM | 291 | HB2 | CYS | 19 | -8.498 | -5.465 | 2.012 | 1.00 | 0.00 |
| ATOM | 292 | C | CYS | 19 | -8.452 | -7.454 | 3.872 | 1.00 | 0.00 |
| ATOM | 293 | O | CYS | 19 | -8.550 | -8.144 | 4.870 | 1.00 | 0.00 |
| ATOM | 294 | CB | CYS | 19 | -8.466 | -5.071 | 3.012 | 1.00 | 0.00 |
| ATOM | 295 | SG | CYS | 19 | -7.549 | -3.504 | 2.992 | 1.00 | 0.00 |
| ATOM | 296 | N | GLY | 20 | -8.933 | -7.860 | 2.718 | 1.00 | 0.00 |
| ATOM | 297 | HN | GLY | 20 | -8.849 | -7.297 | 1.925 | 1.00 | 0.00 |
| ATOM | 298 | CA | GLY | 20 | -9.613 | -9.186 | 2.613 | 1.00 | 0.00 |
| ATOM | 299 | HA1 | GLY | 20 | -10.029 | -9.451 | 3.574 | 1.00 | 0.00 |
| ATOM | 300 | HA2 | GLY | 20 | -8.895 | -9.935 | 2.311 | 1.00 | 0.00 |
| ATOM | 301 | C | GLY | 20 | -10.741 | -9.110 | 1.580 | 1.00 | 0.00 |
| ATOM | 302 | O | GLY | 20 | -10.548 | -8.649 | 0.472 | 1.00 | 0.00 |
| ATOM | 303 | N | GLU | 21 | -11.914 | -9.569 | 1.937 | 1.00 | 0.00 |
| ATOM | 304 | HN | GLU | 21 | -12.038 | -9.939 | 2.837 | 1.00 | 0.00 |
| ATOM | 305 | CA | GLU | 21 | -13.064 | -9.539 | 0.983 | 1.00 | 0.00 |
| ATOM | 306 | HA | GLU | 21 | -12.693 | -9.624 | -0.028 | 1.00 | 0.00 |
| ATOM | 307 | CB | GLU | 21 | -14.004 | -10.713 | 1.283 | 1.00 | 0.00 |
| ATOM | 308 | HB1 | GLU | 21 | -15.030 | -10.392 | 1.186 | 1.00 | 0.00 |
| ATOM | 309 | HB2 | GLU | 21 | -13.831 | -11.061 | 2.292 | 1.00 | 0.00 |
| ATOM | 310 | CG | GLU | 21 | -13.736 | -11.855 | 0.299 | 1.00 | 0.00 |
| ATOM | 311 | HG1 | GLU | 21 | -13.882 | -12.801 | 0.797 | 1.00 | 0.00 |
| ATOM | 312 | HG2 | GLU | 21 | -12.719 | -11.790 | -0.060 | 1.00 | 0.00 |
| ATOM | 313 | CD | GLU | 21 | -14.702 | -11.748 | -0.882 | 1.00 | 0.00 |
| ATOM | 314 | OE1 | GLU | 21 | -14.275 | -11.299 | -1.932 | 1.00 | 0.00 |
| ATOM | 315 | OE2 | GLU | 21 | -15.853 | -12.118 | -0.716 | 1.00 | 0.00 |
| ATOM | 316 | C | GLU | 21 | -13.835 | -8.223 | 1.132 | 1.00 | 0.00 |
| ATOM | 317 | O | GLU | 21 | -14.459 | -7.753 | 0.199 | 1.00 | 0.00 |
| ATOM | 318 | N | ARG | 22 | -13.808 | -7.631 | 2.302 | 1.00 | 0.00 |
| ATOM | 319 | HN | ARG | 22 | -13.304 | -8.035 | 3.040 | 1.00 | 0.00 |
| ATOM | 320 | CA | ARG | 22 | -14.548 | -6.350 | 2.524 | 1.00 | 0.00 |
| ATOM | 321 | HA | ARG | 22 | -15.605 | -6.522 | 2.398 | 1.00 | 0.00 |
| ATOM | 322 | CB | ARG | 22 | -14.288 | -5.853 | 3.944 | 1.00 | 0.00 |
| ATOM | 323 | HB1 | ARG | 22 | -14.444 | -4.788 | 3.983 | 1.00 | 0.00 |
| ATOM | 324 | HB2 | ARG | 22 | -13.269 | -6.080 | 4.225 | 1.00 | 0.00 |
| ATOM | 325 | CG | ARG | 22 | -15.251 | -6.543 | 4.912 | 1.00 | 0.00 |
| ATOM | 326 | HG1 | ARG | 22 | -14.802 | -7.452 | 5.281 | 1.00 | 0.00 |
| ATOM | 327 | HG2 | ARG | 22 | -16.172 | -6.778 | 4.395 | 1.00 | 0.00 |
| ATOM | 328 | CD | ARG | 22 | -15.549 | -5.610 | 6.085 | 1.00 | 0.00 |
| ATOM | 329 | HD1 | ARG | 22 | -16.250 | -6.085 | 6.755 | 1.00 | 0.00 |
| ATOM | 330 | HD2 | ARG | 22 | -15.973 | -4.689 | 5.714 | 1.00 | 0.00 |
| ATOM | 331 | NE | ARG | 22 | -14.285 | -5.315 | 6.818 | 1.00 | 0.00 |
| ATOM | 332 | HE | ARG | 22 | -13.502 | -4.985 | 6.331 | 1.00 | 0.00 |
| ATOM | 333 | CZ | ARG | 22 | -14.224 | -5.496 | 8.107 | 1.00 | 0.00 |
| ATOM | 334 | NH1 | ARG | 22 | -13.711 | -6.595 | 8.589 | 1.00 | 0.00 |
| ATOM | 335 | HH11 | ARG | 22 | -13.363 | -7.298 | 7.968 | 1.00 | 0.00 |
| ATOM | 336 | HH12 | ARG | 22 | -13.666 | -6.735 | 9.578 | 1.00 | 0.00 |
| ATOM | 337 | NH2 | ARG | 22 | -14.679 | -4.579 | 8.917 | 1.00 | 0.00 |
| ATOM | 338 | HH21 | ARG | 22 | -15.074 | -3.737 | 8.547 | 1.00 | 0.00 |
| ATOM | 339 | HH22 | ARG | 22 | -14.634 | -4.717 | 9.905 | 1.00 | 0.00 |
| ATOM | 340 | C | ARG | 22 | -14.086 | -5.290 | 1.520 | 1.00 | 0.00 |
| ATOM | 341 | O | ARG | 22 | -14.890 | -4.676 | 0.843 | 1.00 | 0.00 |
| ATOM | 342 | N | GLY | 23 | -12.799 | -5.074 | 1.417 | 1.00 | 0.00 |
| ATOM | 343 | HN | GLY | 23 | -12.173 | -5.585 | 1.972 | 1.00 | 0.00 |
| ATOM | 344 | CA | GLY | 23 | -12.281 | -4.057 | 0.456 | 1.00 | 0.00 |
| ATOM | 345 | HA1 | GLY | 23 | -13.077 | -3.381 | 0.182 | 1.00 | 0.00 |
| ATOM | 346 | HA2 | GLY | 23 | -11.913 | -4.556 | -0.429 | 1.00 | 0.00 |
| ATOM | 347 | C | GLY | 23 | -11.146 | -3.266 | 1.102 | 1.00 | 0.00 |
| ATOM | 348 | O | GLY | 23 | -10.816 | -3.465 | 2.256 | 1.00 | 0.00 |
| ATOM | 349 | N | PHE | 24 | -10.547 | -2.370 | 0.361 | 1.00 | 0.00 |
| ATOM | 350 | HN | PHE | 24 | -10.835 | -2.236 | -0.565 | 1.00 | 0.00 |
| ATOM | 351 | CA | PHE | 24 | -9.425 | -1.556 | 0.914 | 1.00 | 0.00 |
| ATOM | 352 | HA | PHE | 24 | -9.307 | -1.785 | 1.957 | 1.00 | 0.00 |
| ATOM | 353 | CB | PHE | 24 | -8.124 | -1.905 | 0.165 | 1.00 | 0.00 |
| ATOM | 354 | HB1 | PHE | 24 | -7.810 | -2.901 | 0.433 | 1.00 | 0.00 |
| ATOM | 355 | HB2 | PHE | 24 | -7.355 | -1.201 | 0.426 | 1.00 | 0.00 |
| ATOM | 356 | CG | PHE | 24 | -8.358 | -1.845 | -1.319 | 1.00 | 0.00 |
| ATOM | 357 | CD1 | PHE | 24 | -8.560 | -0.612 | -1.925 | 1.00 | 0.00 |
| ATOM | 358 | HD1 | PHE | 24 | -8.529 | 0.279 | -1.325 | 1.00 | 0.00 |
| ATOM | 359 | CD2 | PHE | 24 | -8.387 | -3.019 | -2.077 | 1.00 | 0.00 |
| ATOM | 360 | HD2 | PHE | 24 | -8.212 | -3.981 | -1.597 | 1.00 | 0.00 |
| ATOM | 361 | CE1 | PHE | 24 | -8.800 | -0.532 | -3.300 | 1.00 | 0.00 |
| ATOM | 362 | HE1 | PHE | 24 | -8.956 | 0.429 | -3.768 | 1.00 | 0.00 |
| ATOM | 363 | CE2 | PHE | 24 | -8.625 | -2.943 | -3.457 | 1.00 | 0.00 |
| ATOM | 364 | HE2 | PHE | 24 | -8.657 | -3.844 | -4.051 | 1.00 | 0.00 |
| ATOM | 365 | CZ | PHE | 24 | -8.833 | -1.700 | -4.068 | 1.00 | 0.00 |
| ATOM | 366 | HZ | PHE | 24 | -9.018 | -1.643 | -5.130 | 1.00 | 0.00 |
| ATOM | 367 | C | PHE | 24 | -9.755 | -0.061 | 0.759 | 1.00 | 0.00 |
| ATOM | 368 | O | PHE | 24 | -10.883 | 0.304 | 0.486 | 1.00 | 0.00 |
| ATOM | 369 | N | PHE | 25 | -8.782 | 0.806 | 0.931 | 1.00 | 0.00 |
| ATOM | 370 | HN | PHE | 25 | -7.883 | 0.493 | 1.152 | 1.00 | 0.00 |
| ATOM | 371 | CA | PHE | 25 | -9.036 | 2.274 | 0.796 | 1.00 | 0.00 |
| ATOM | 372 | HA | PHE | 25 | -10.093 | 2.450 | 0.662 | 1.00 | 0.00 |
| ATOM | 373 | CB | PHE | 25 | -8.553 | 2.987 | 2.063 | 1.00 | 0.00 |
| ATOM | 374 | HB1 | PHE | 25 | -8.158 | 3.958 | 1.800 | 1.00 | 0.00 |
| ATOM | 375 | HB2 | PHE | 25 | -7.776 | 2.401 | 2.522 | 1.00 | 0.00 |
| ATOM | 376 | CG | PHE | 25 | -9.693 | 3.162 | 3.040 | 1.00 | 0.00 |
| ATOM | 377 | CD1 | PHE | 25 | -9.908 | 4.410 | 3.637 | 1.00 | 0.00 |
| ATOM | 378 | HD1 | PHE | 25 | -9.267 | 5.244 | 3.392 | 1.00 | 0.00 |
| ATOM | 379 | CD2 | PHE | 25 | -10.524 | 2.082 | 3.360 | 1.00 | 0.00 |
| ATOM | 380 | HD2 | PHE | 25 | -10.359 | 1.119 | 2.900 | 1.00 | 0.00 |
| ATOM | 381 | CE1 | PHE | 25 | -10.955 | 4.579 | 4.550 | 1.00 | 0.00 |
| ATOM | 382 | HE1 | PHE | 25 | -11.118 | 5.542 | 5.010 | 1.00 | 0.00 |
| ATOM | 383 | CE2 | PHE | 25 | -11.570 | 2.252 | 4.271 | 1.00 | 0.00 |
| ATOM | 384 | HE2 | PHE | 25 | -12.210 | 1.421 | 4.514 | 1.00 | 0.00 |
| ATOM | 385 | CZ | PHE | 25 | -11.787 | 3.500 | 4.867 | 1.00 | 0.00 |
| ATOM | 386 | HZ | PHE | 25 | -12.594 | 3.629 | 5.572 | 1.00 | 0.00 |
| ATOM | 387 | C | PHE | 25 | -8.263 | 2.828 | -0.409 | 1.00 | 0.00 |
| ATOM | 388 | O | PHE | 25 | -7.421 | 2.161 | -0.979 | 1.00 | 0.00 |
| ATOM | 389 | N | TYR | 26 | -8.540 | 4.051 | -0.784 | 1.00 | 0.00 |
| ATOM | 390 | HN | TYR | 26 | -9.216 | 4.567 | -0.297 | 1.00 | 0.00 |
| ATOM | 391 | CA | TYR | 26 | -7.825 | 4.671 | -1.938 | 1.00 | 0.00 |
| ATOM | 392 | HA | TYR | 26 | -6.785 | 4.381 | -1.915 | 1.00 | 0.00 |
| ATOM | 393 | CB | TYR | 26 | -8.463 | 4.196 | -3.248 | 1.00 | 0.00 |
| ATOM | 394 | HB1 | TYR | 26 | -9.171 | 4.934 | -3.593 | 1.00 | 0.00 |
| ATOM | 395 | HB2 | TYR | 26 | -8.975 | 3.259 | -3.078 | 1.00 | 0.00 |
| ATOM | 396 | CG | TYR | 26 | -7.391 | 4.000 | -4.295 | 1.00 | 0.00 |
| ATOM | 397 | CD1 | TYR | 26 | -7.086 | 5.034 | -5.188 | 1.00 | 0.00 |
| ATOM | 398 | HD1 | TYR | 26 | -7.617 | 5.973 | -5.129 | 1.00 | 0.00 |
| ATOM | 399 | CD2 | TYR | 26 | -6.701 | 2.783 | -4.373 | 1.00 | 0.00 |
| ATOM | 400 | HD2 | TYR | 26 | -6.936 | 1.985 | -3.684 | 1.00 | 0.00 |
| ATOM | 401 | CE1 | TYR | 26 | -6.093 | 4.852 | -6.159 | 1.00 | 0.00 |
| ATOM | 402 | HE1 | TYR | 26 | -5.858 | 5.650 | -6.847 | 1.00 | 0.00 |
| ATOM | 403 | CE2 | TYR | 26 | -5.708 | 2.602 | -5.344 | 1.00 | 0.00 |
| ATOM | 404 | HE2 | TYR | 26 | -5.177 | 1.663 | -5.404 | 1.00 | 0.00 |
| ATOM | 405 | CZ | TYR | 26 | -5.403 | 3.637 | -6.236 | 1.00 | 0.00 |
| ATOM | 406 | OH | TYR | 26 | -4.425 | 3.459 | -7.192 | 1.00 | 0.00 |
| ATOM | 407 | HH | TYR | 26 | -4.787 | 2.908 | -7.890 | 1.00 | 0.00 |
| ATOM | 408 | C | TYR | 26 | -7.934 | 6.195 | -1.835 | 1.00 | 0.00 |
| ATOM | 409 | O | TYR | 26 | -8.901 | 6.787 | -2.277 | 1.00 | 0.00 |
| ATOM | 410 | N | THR | 27 | -6.953 | 6.830 | -1.241 | 1.00 | 0.00 |
| ATOM | 411 | HN | THR | 27 | -6.191 | 6.325 | -0.888 | 1.00 | 0.00 |
| ATOM | 412 | CA | THR | 27 | -6.994 | 8.316 | -1.090 | 1.00 | 0.00 |
| ATOM | 413 | HA | THR | 27 | -7.965 | 8.612 | -0.720 | 1.00 | 0.00 |
| ATOM | 414 | CB | THR | 27 | -5.915 | 8.758 | -0.091 | 1.00 | 0.00 |
| ATOM | 415 | HB | THR | 27 | -6.127 | 8.330 | 0.876 | 1.00 | 0.00 |
| ATOM | 416 | OG1 | THR | 27 | -5.920 | 10.175 | 0.008 | 1.00 | 0.00 |
| ATOM | 417 | HG1 | THR | 27 | -6.818 | 10.458 | 0.194 | 1.00 | 0.00 |
| ATOM | 418 | CG2 | THR | 27 | -4.535 | 8.282 | -0.562 | 1.00 | 0.00 |
| ATOM | 419 | HG21 | THR | 27 | -4.643 | 7.648 | -1.431 | 1.00 | 0.00 |
| ATOM | 420 | HG22 | THR | 27 | -4.058 | 7.725 | 0.231 | 1.00 | 0.00 |
| ATOM | 421 | HG23 | THR | 27 | -3.926 | 9.137 | -0.814 | 1.00 | 0.00 |
| ATOM | 422 | C | THR | 27 | -6.743 | 8.988 | -2.442 | 1.00 | 0.00 |
| ATOM | 423 | O | THR | 27 | -6.319 | 8.357 | -3.392 | 1.00 | 0.00 |
| ATOM | 424 | N | ASP | 28 | -6.999 | 10.268 | -2.527 | 1.00 | 0.00 |
| ATOM | 425 | HN | ASP | 28 | -7.337 | 10.750 | -1.743 | 1.00 | 0.00 |
| ATOM | 426 | CA | ASP | 28 | -6.779 | 11.004 | -3.806 | 1.00 | 0.00 |
| ATOM | 427 | HA | ASP | 28 | -5.809 | 10.748 | -4.207 | 1.00 | 0.00 |
| ATOM | 428 | CB | ASP | 28 | -7.870 | 10.622 | -4.812 | 1.00 | 0.00 |
| ATOM | 429 | HB1 | ASP | 28 | -8.163 | 11.495 | -5.377 | 1.00 | 0.00 |
| ATOM | 430 | HB2 | ASP | 28 | -8.726 | 10.231 | -4.281 | 1.00 | 0.00 |
| ATOM | 431 | CG | ASP | 28 | -7.333 | 9.556 | -5.771 | 1.00 | 0.00 |
| ATOM | 432 | OD1 | ASP | 28 | -6.648 | 9.924 | -6.711 | 1.00 | 0.00 |
| ATOM | 433 | OD2 | ASP | 28 | -7.617 | 8.390 | -5.549 | 1.00 | 0.00 |
| ATOM | 434 | C | ASP | 28 | -6.837 | 12.509 | -3.534 | 1.00 | 0.00 |
| ATOM | 435 | O | ASP | 28 | -5.850 | 13.209 | -3.651 | 1.00 | 0.00 |
| ATOM | 436 | N | LYS | 29 | -7.992 | 13.007 | -3.169 | 1.00 | 0.00 |
| ATOM | 437 | HN | LYS | 29 | -8.770 | 12.418 | -3.083 | 1.00 | 0.00 |
| ATOM | 438 | CA | LYS | 29 | -8.126 | 14.466 | -2.885 | 1.00 | 0.00 |
| ATOM | 439 | HA | LYS | 29 | -7.666 | 15.029 | -3.684 | 1.00 | 0.00 |
| ATOM | 440 | CB | LYS | 29 | -9.614 | 14.835 | -2.798 | 1.00 | 0.00 |
| ATOM | 441 | HB1 | LYS | 29 | -10.092 | 14.617 | -3.741 | 1.00 | 0.00 |
| ATOM | 442 | HB2 | LYS | 29 | -9.708 | 15.890 | -2.585 | 1.00 | 0.00 |
| ATOM | 443 | CG | LYS | 29 | -10.293 | 14.027 | -1.685 | 1.00 | 0.00 |
| ATOM | 444 | HG1 | LYS | 29 | -10.315 | 14.614 | -0.779 | 1.00 | 0.00 |
| ATOM | 445 | HG2 | LYS | 29 | -9.741 | 13.116 | -1.510 | 1.00 | 0.00 |
| ATOM | 446 | CD | LYS | 29 | -11.725 | 13.685 | -2.101 | 1.00 | 0.00 |
| ATOM | 447 | HD1 | LYS | 29 | -11.719 | 12.806 | -2.728 | 1.00 | 0.00 |
| ATOM | 448 | HD2 | LYS | 29 | -12.149 | 14.515 | -2.648 | 1.00 | 0.00 |
| ATOM | 449 | CE | LYS | 29 | -12.565 | 13.413 | -0.853 | 1.00 | 0.00 |
| ATOM | 450 | HE1 | LYS | 29 | -13.613 | 13.525 | -1.093 | 1.00 | 0.00 |
| ATOM | 451 | HE2 | LYS | 29 | -12.297 | 14.115 | -0.078 | 1.00 | 0.00 |
| ATOM | 452 | NZ | LYS | 29 | -12.311 | 12.024 | -0.375 | 1.00 | 0.00 |
| ATOM | 453 | HZ1 | LYS | 29 | -11.297 | 11.810 | -0.453 | 1.00 | 0.00 |
| ATOM | 454 | HZ2 | LYS | 29 | -12.854 | 11.353 | -0.957 | 1.00 | 0.00 |
| ATOM | 455 | HZ3 | LYS | 29 | -12.605 | 11.939 | 0.618 | 1.00 | 0.00 |
| ATOM | 456 | C | LYS | 29 | -7.429 | 14.801 | -1.562 | 1.00 | 0.00 |
| ATOM | 457 | O | LYS | 29 | -6.940 | 15.899 | -1.369 | 1.00 | 0.00 |
| ATOM | 458 | N | ASP | 30 | -7.387 | 13.862 | -0.651 | 1.00 | 0.00 |
| ATOM | 459 | HN | ASP | 30 | -7.793 | 12.989 | -0.833 | 1.00 | 0.00 |
| ATOM | 460 | CA | ASP | 30 | -6.730 | 14.112 | 0.667 | 1.00 | 0.00 |
| ATOM | 461 | HA | ASP | 30 | -7.187 | 14.969 | 1.138 | 1.00 | 0.00 |
| ATOM | 462 | CB | ASP | 30 | -6.905 | 12.884 | 1.562 | 1.00 | 0.00 |
| ATOM | 463 | HB1 | ASP | 30 | -6.222 | 12.109 | 1.249 | 1.00 | 0.00 |
| ATOM | 464 | HB2 | ASP | 30 | -7.920 | 12.523 | 1.481 | 1.00 | 0.00 |
| ATOM | 465 | CG | ASP | 30 | -6.612 | 13.262 | 3.016 | 1.00 | 0.00 |
| ATOM | 466 | OD1 | ASP | 30 | -6.960 | 14.367 | 3.402 | 1.00 | 0.00 |
| ATOM | 467 | OD2 | ASP | 30 | -6.045 | 12.442 | 3.719 | 1.00 | 0.00 |
| ATOM | 468 | C | ASP | 30 | -5.238 | 14.384 | 0.456 | 1.00 | 0.00 |
| ATOM | 469 | O | ASP | 30 | -4.735 | 15.434 | 0.811 | 1.00 | 0.00 |
| ATOM | 470 | N | GLY | 31 | -4.526 | 13.444 | -0.116 | 1.00 | 0.00 |
| ATOM | 471 | HN | GLY | 31 | -4.956 | 12.607 | -0.392 | 1.00 | 0.00 |
| ATOM | 472 | CA | GLY | 31 | -3.066 | 13.643 | -0.351 | 1.00 | 0.00 |
| ATOM | 473 | HA1 | GLY | 31 | -2.510 | 12.870 | 0.158 | 1.00 | 0.00 |
| ATOM | 474 | HA2 | GLY | 31 | -2.769 | 14.611 | 0.028 | 1.00 | 0.00 |
| ATOM | 475 | C | GLY | 31 | -2.772 | 13.569 | -1.851 | 1.00 | 0.00 |
| ATOM | 476 | O | GLY | 31 | -3.342 | 14.300 | -2.639 | 1.00 | 0.00 |
| ATOM | 477 | N | LYS | 32 | -1.887 | 12.690 | -2.246 | 1.00 | 0.00 |
| ATOM | 478 | HN | LYS | 32 | -1.445 | 12.114 | -1.589 | 1.00 | 0.00 |
| ATOM | 479 | CA | LYS | 32 | -1.550 | 12.559 | -3.694 | 1.00 | 0.00 |
| ATOM | 480 | HA | LYS | 32 | -2.213 | 13.182 | -4.274 | 1.00 | 0.00 |
| ATOM | 481 | CB | LYS | 32 | -0.102 | 13.003 | -3.922 | 1.00 | 0.00 |
| ATOM | 482 | HB1 | LYS | 32 | 0.545 | 12.139 | -3.924 | 1.00 | 0.00 |
| ATOM | 483 | HB2 | LYS | 32 | 0.198 | 13.675 | -3.131 | 1.00 | 0.00 |
| ATOM | 484 | CG | LYS | 32 | 0.004 | 13.719 | -5.269 | 1.00 | 0.00 |
| ATOM | 485 | HG1 | LYS | 32 | -0.951 | 14.154 | -5.522 | 1.00 | 0.00 |
| ATOM | 486 | HG2 | LYS | 32 | 0.290 | 13.010 | -6.032 | 1.00 | 0.00 |
| ATOM | 487 | CD | LYS | 32 | 1.058 | 14.827 | -5.180 | 1.00 | 0.00 |
| ATOM | 488 | HD1 | LYS | 32 | 2.023 | 14.427 | -5.452 | 1.00 | 0.00 |
| ATOM | 489 | HD2 | LYS | 32 | 1.097 | 15.203 | -4.168 | 1.00 | 0.00 |
| ATOM | 490 | CE | LYS | 32 | 0.695 | 15.967 | -6.137 | 1.00 | 0.00 |
| ATOM | 491 | HE1 | LYS | 32 | -0.301 | 15.816 | -6.527 | 1.00 | 0.00 |
| ATOM | 492 | HE2 | LYS | 32 | 1.401 | 15.990 | -6.953 | 1.00 | 0.00 |
| ATOM | 493 | NZ | LYS | 32 | 0.746 | 17.263 | -5.402 | 1.00 | 0.00 |
| ATOM | 494 | HZ1 | LYS | 32 | 1.615 | 17.304 | -4.833 | 1.00 | 0.00 |
| ATOM | 495 | HZ2 | LYS | 32 | -0.083 | 17.339 | -4.777 | 1.00 | 0.00 |
| ATOM | 496 | HZ3 | LYS | 32 | 0.741 | 18.048 | -6.082 | 1.00 | 0.00 |
| ATOM | 497 | C | LYS | 32 | -1.712 | 11.100 | -4.127 | 1.00 | 0.00 |
| ATOM | 498 | O | LYS | 32 | -1.028 | 10.629 | -5.015 | 1.00 | 0.00 |
| ATOM | 499 | N | GLY | 33 | -2.614 | 10.385 | -3.504 | 1.00 | 0.00 |
| ATOM | 500 | HN | GLY | 33 | -3.152 | 10.792 | -2.792 | 1.00 | 0.00 |
| ATOM | 501 | CA | GLY | 33 | -2.831 | 8.956 | -3.872 | 1.00 | 0.00 |
| ATOM | 502 | HA1 | GLY | 33 | -2.770 | 8.848 | -4.945 | 1.00 | 0.00 |
| ATOM | 503 | HA2 | GLY | 33 | -3.808 | 8.643 | -3.532 | 1.00 | 0.00 |
| ATOM | 504 | C | GLY | 33 | -1.758 | 8.085 | -3.216 | 1.00 | 0.00 |
| ATOM | 505 | O | GLY | 33 | -0.590 | 8.419 | -3.217 | 1.00 | 0.00 |
| ATOM | 506 | N | ILE | 34 | -2.151 | 6.971 | -2.656 | 1.00 | 0.00 |
| ATOM | 507 | HN | ILE | 34 | -3.101 | 6.728 | -2.667 | 1.00 | 0.00 |
| ATOM | 508 | CA | ILE | 34 | -1.166 | 6.063 | -1.993 | 1.00 | 0.00 |
| ATOM | 509 | HA | ILE | 34 | -0.651 | 6.600 | -1.211 | 1.00 | 0.00 |
| ATOM | 510 | CB | ILE | 34 | -1.905 | 4.872 | -1.389 | 1.00 | 0.00 |
| ATOM | 511 | HB | ILE | 34 | -1.184 | 4.158 | -1.016 | 1.00 | 0.00 |
| ATOM | 512 | CG1 | ILE | 34 | -2.780 | 4.208 | -2.463 | 1.00 | 0.00 |
| ATOM | 513 | HG11 | ILE | 34 | -2.453 | 4.521 | -3.443 | 1.00 | 0.00 |
| ATOM | 514 | HG12 | ILE | 34 | -3.812 | 4.497 | -2.320 | 1.00 | 0.00 |
| ATOM | 515 | CG2 | ILE | 34 | -2.783 | 5.348 | -0.237 | 1.00 | 0.00 |
| ATOM | 516 | HG21 | ILE | 34 | -3.214 | 4.491 | 0.255 | 1.00 | 0.00 |
| ATOM | 517 | HG22 | ILE | 34 | -3.572 | 5.977 | -0.621 | 1.00 | 0.00 |
| ATOM | 518 | HG23 | ILE | 34 | -2.185 | 5.906 | 0.466 | 1.00 | 0.00 |
| ATOM | 519 | CD1 | ILE | 34 | -2.660 | 2.686 | -2.356 | 1.00 | 0.00 |
| ATOM | 520 | HD11 | ILE | 34 | -3.421 | 2.221 | -2.965 | 1.00 | 0.00 |
| ATOM | 521 | HD12 | ILE | 34 | -2.791 | 2.387 | -1.326 | 1.00 | 0.00 |
| ATOM | 522 | HD13 | ILE | 34 | -1.685 | 2.376 | -2.700 | 1.00 | 0.00 |
| ATOM | 523 | C | ILE | 34 | -0.146 | 5.536 | -3.011 | 1.00 | 0.00 |
| ATOM | 524 | O | ILE | 34 | 0.905 | 5.052 | -2.641 | 1.00 | 0.00 |
| ATOM | 525 | N | VAL | 35 | -0.460 | 5.593 | -4.280 | 1.00 | 0.00 |
| ATOM | 526 | HN | VAL | 35 | -1.324 | 5.962 | -4.555 | 1.00 | 0.00 |
| ATOM | 527 | CA | VAL | 35 | 0.479 | 5.063 | -5.316 | 1.00 | 0.00 |
| ATOM | 528 | HA | VAL | 35 | 0.679 | 4.022 | -5.111 | 1.00 | 0.00 |
| ATOM | 529 | CB | VAL | 35 | -0.172 | 5.185 | -6.695 | 1.00 | 0.00 |
| ATOM | 530 | HB | VAL | 35 | -0.280 | 6.229 | -6.951 | 1.00 | 0.00 |
| ATOM | 531 | CG1 | VAL | 35 | 0.706 | 4.491 | -7.739 | 1.00 | 0.00 |
| ATOM | 532 | HG11 | VAL | 35 | 0.080 | 4.041 | -8.496 | 1.00 | 0.00 |
| ATOM | 533 | HG12 | VAL | 35 | 1.299 | 3.725 | -7.259 | 1.00 | 0.00 |
| ATOM | 534 | HG13 | VAL | 35 | 1.360 | 5.218 | -8.198 | 1.00 | 0.00 |
| ATOM | 535 | CG2 | VAL | 35 | -1.551 | 4.518 | -6.665 | 1.00 | 0.00 |
| ATOM | 536 | HG21 | VAL | 35 | -1.505 | 3.626 | -6.058 | 1.00 | 0.00 |
| ATOM | 537 | HG22 | VAL | 35 | -1.847 | 4.255 | -7.669 | 1.00 | 0.00 |
| ATOM | 538 | HG23 | VAL | 35 | -2.273 | 5.204 | -6.245 | 1.00 | 0.00 |
| ATOM | 539 | C | VAL | 35 | 1.802 | 5.840 | -5.303 | 1.00 | 0.00 |
| ATOM | 540 | O | VAL | 35 | 2.820 | 5.331 | -5.726 | 1.00 | 0.00 |
| ATOM | 541 | N | GLU | 36 | 1.799 | 7.063 | -4.833 | 1.00 | 0.00 |
| ATOM | 542 | HN | GLU | 36 | 0.965 | 7.458 | -4.504 | 1.00 | 0.00 |
| ATOM | 543 | CA | GLU | 36 | 3.066 | 7.861 | -4.814 | 1.00 | 0.00 |
| ATOM | 544 | HA | GLU | 36 | 3.623 | 7.664 | -5.718 | 1.00 | 0.00 |
| ATOM | 545 | CB | GLU | 36 | 2.737 | 9.359 | -4.745 | 1.00 | 0.00 |
| ATOM | 546 | HB1 | GLU | 36 | 2.149 | 9.635 | -5.608 | 1.00 | 0.00 |
| ATOM | 547 | HB2 | GLU | 36 | 3.657 | 9.926 | -4.745 | 1.00 | 0.00 |
| ATOM | 548 | CG | GLU | 36 | 1.940 | 9.671 | -3.469 | 1.00 | 0.00 |
| ATOM | 549 | HG1 | GLU | 36 | 1.907 | 8.794 | -2.840 | 1.00 | 0.00 |
| ATOM | 550 | HG2 | GLU | 36 | 0.934 | 9.957 | -3.739 | 1.00 | 0.00 |
| ATOM | 551 | CD | GLU | 36 | 2.607 | 10.817 | -2.704 | 1.00 | 0.00 |
| ATOM | 552 | OE1 | GLU | 36 | 2.693 | 11.902 | -3.257 | 1.00 | 0.00 |
| ATOM | 553 | OE2 | GLU | 36 | 3.017 | 10.591 | -1.577 | 1.00 | 0.00 |
| ATOM | 554 | C | GLU | 36 | 3.931 | 7.469 | -3.607 | 1.00 | 0.00 |
| ATOM | 555 | O | GLU | 36 | 5.117 | 7.727 | -3.586 | 1.00 | 0.00 |
| ATOM | 556 | N | GLN | 37 | 3.350 | 6.870 | -2.599 | 1.00 | 0.00 |
| ATOM | 557 | HN | GLN | 37 | 2.392 | 6.686 | -2.626 | 1.00 | 0.00 |
| ATOM | 558 | CA | GLN | 37 | 4.148 | 6.481 | -1.397 | 1.00 | 0.00 |
| ATOM | 559 | HA | GLN | 37 | 4.977 | 7.165 | -1.285 | 1.00 | 0.00 |
| ATOM | 560 | CB | GLN | 37 | 3.261 | 6.559 | -0.153 | 1.00 | 0.00 |
| ATOM | 561 | HB1 | GLN | 37 | 2.696 | 5.644 | -0.056 | 1.00 | 0.00 |
| ATOM | 562 | HB2 | GLN | 37 | 2.583 | 7.396 | -0.247 | 1.00 | 0.00 |
| ATOM | 563 | CG | GLN | 37 | 4.141 | 6.748 | 1.085 | 1.00 | 0.00 |
| ATOM | 564 | HG1 | GLN | 37 | 4.996 | 7.357 | 0.829 | 1.00 | 0.00 |
| ATOM | 565 | HG2 | GLN | 37 | 4.479 | 5.783 | 1.435 | 1.00 | 0.00 |
| ATOM | 566 | CD | GLN | 37 | 3.337 | 7.438 | 2.188 | 1.00 | 0.00 |
| ATOM | 567 | OE1 | GLN | 37 | 3.428 | 8.637 | 2.363 | 1.00 | 0.00 |
| ATOM | 568 | NE2 | GLN | 37 | 2.549 | 6.726 | 2.946 | 1.00 | 0.00 |
| ATOM | 569 | HE21 | GLN | 37 | 2.476 | 5.760 | 2.806 | 1.00 | 0.00 |
| ATOM | 570 | HE22 | GLN | 37 | 2.033 | 7.159 | 3.658 | 1.00 | 0.00 |
| ATOM | 571 | C | GLN | 37 | 4.689 | 5.053 | -1.548 | 1.00 | 0.00 |
| ATOM | 572 | O | GLN | 37 | 5.778 | 4.749 | -1.099 | 1.00 | 0.00 |
| ATOM | 573 | N | CYS | 38 | 3.930 | 4.169 | -2.150 | 1.00 | 0.00 |
| ATOM | 574 | HN | CYS | 38 | 3.049 | 4.430 | -2.488 | 1.00 | 0.00 |
| ATOM | 575 | CA | CYS | 38 | 4.396 | 2.753 | -2.299 | 1.00 | 0.00 |
| ATOM | 576 | HA | CYS | 38 | 5.053 | 2.514 | -1.480 | 1.00 | 0.00 |
| ATOM | 577 | HB1 | CYS | 38 | 3.526 | 0.793 | -2.375 | 1.00 | 0.00 |
| ATOM | 578 | HB2 | CYS | 38 | 2.520 | 2.059 | -3.077 | 1.00 | 0.00 |
| ATOM | 579 | C | CYS | 38 | 5.164 | 2.558 | -3.610 | 1.00 | 0.00 |
| ATOM | 580 | O | CYS | 38 | 6.166 | 1.873 | -3.639 | 1.00 | 0.00 |
| ATOM | 581 | CB | CYS | 38 | 3.188 | 1.813 | -2.264 | 1.00 | 0.00 |
| ATOM | 582 | SG | CYS | 38 | 2.305 | 1.990 | -0.686 | 1.00 | 0.00 |
| ATOM | 583 | N | CYS | 39 | 4.717 | 3.135 | -4.695 | 1.00 | 0.00 |
| ATOM | 584 | HN | CYS | 39 | 3.906 | 3.683 | -4.666 | 1.00 | 0.00 |
| ATOM | 585 | CA | CYS | 39 | 5.459 | 2.950 | -5.982 | 1.00 | 0.00 |
| ATOM | 586 | HA | CYS | 39 | 5.633 | 1.897 | -6.130 | 1.00 | 0.00 |
| ATOM | 587 | HB1 | CYS | 39 | 4.453 | 4.551 | -6.994 | 1.00 | 0.00 |
| ATOM | 588 | HB2 | CYS | 39 | 3.678 | 2.985 | -7.182 | 1.00 | 0.00 |
| ATOM | 589 | C | CYS | 39 | 6.813 | 3.671 | -5.901 | 1.00 | 0.00 |
| ATOM | 590 | O | CYS | 39 | 7.723 | 3.370 | -6.650 | 1.00 | 0.00 |
| ATOM | 591 | CB | CYS | 39 | 4.626 | 3.499 | -7.149 | 1.00 | 0.00 |
| ATOM | 592 | SG | CYS | 39 | 5.492 | 3.260 | -8.732 | 1.00 | 0.00 |
| ATOM | 593 | N | THR | 40 | 6.958 | 4.611 | -4.994 | 1.00 | 0.00 |
| ATOM | 594 | HN | THR | 40 | 6.216 | 4.837 | -4.397 | 1.00 | 0.00 |
| ATOM | 595 | CA | THR | 40 | 8.256 | 5.337 | -4.866 | 1.00 | 0.00 |
| ATOM | 596 | HA | THR | 40 | 8.733 | 5.391 | -5.833 | 1.00 | 0.00 |
| ATOM | 597 | CB | THR | 40 | 8.000 | 6.751 | -4.343 | 1.00 | 0.00 |
| ATOM | 598 | HB | THR | 40 | 8.940 | 7.258 | -4.199 | 1.00 | 0.00 |
| ATOM | 599 | OG1 | THR | 40 | 7.305 | 6.680 | -3.107 | 1.00 | 0.00 |
| ATOM | 600 | HG1 | THR | 40 | 7.327 | 7.550 | -2.702 | 1.00 | 0.00 |
| ATOM | 601 | CG2 | THR | 40 | 7.164 | 7.527 | -5.364 | 1.00 | 0.00 |
| ATOM | 602 | HG21 | THR | 40 | 7.651 | 7.496 | -6.326 | 1.00 | 0.00 |
| ATOM | 603 | HG22 | THR | 40 | 7.066 | 8.554 | -5.042 | 1.00 | 0.00 |
| ATOM | 604 | HG23 | THR | 40 | 6.183 | 7.080 | -5.442 | 1.00 | 0.00 |
| ATOM | 605 | C | THR | 40 | 9.166 | 4.582 | -3.891 | 1.00 | 0.00 |
| ATOM | 606 | O | THR | 40 | 10.293 | 4.253 | -4.215 | 1.00 | 0.00 |
| ATOM | 607 | N | SER | 41 | 8.686 | 4.300 | -2.702 | 1.00 | 0.00 |
| ATOM | 608 | HN | SER | 41 | 7.775 | 4.572 | -2.467 | 1.00 | 0.00 |
| ATOM | 609 | CA | SER | 41 | 9.524 | 3.562 | -1.709 | 1.00 | 0.00 |
| ATOM | 610 | HA | SER | 41 | 10.499 | 3.375 | -2.134 | 1.00 | 0.00 |
| ATOM | 611 | CB | SER | 41 | 9.670 | 4.394 | -0.435 | 1.00 | 0.00 |
| ATOM | 612 | HB1 | SER | 41 | 8.877 | 4.137 | 0.255 | 1.00 | 0.00 |
| ATOM | 613 | HB2 | SER | 41 | 9.604 | 5.441 | -0.679 | 1.00 | 0.00 |
| ATOM | 614 | OG | SER | 41 | 10.936 | 4.128 | 0.153 | 1.00 | 0.00 |
| ATOM | 615 | HG | SER | 41 | 10.794 | 3.925 | 1.080 | 1.00 | 0.00 |
| ATOM | 616 | C | SER | 41 | 8.860 | 2.225 | -1.377 | 1.00 | 0.00 |
| ATOM | 617 | O | SER | 41 | 7.796 | 1.921 | -1.867 | 1.00 | 0.00 |
| ATOM | 618 | N | ILE | 42 | 9.489 | 1.418 | -0.567 | 1.00 | 0.00 |
| ATOM | 619 | HN | ILE | 42 | 10.357 | 1.676 | -0.193 | 1.00 | 0.00 |
| ATOM | 620 | CA | ILE | 42 | 8.897 | 0.088 | -0.235 | 1.00 | 0.00 |
| ATOM | 621 | HA | ILE | 42 | 8.391 | -0.300 | -1.106 | 1.00 | 0.00 |
| ATOM | 622 | CB | ILE | 42 | 10.011 | -0.883 | 0.172 | 1.00 | 0.00 |
| ATOM | 623 | HB | ILE | 42 | 10.391 | -0.610 | 1.147 | 1.00 | 0.00 |
| ATOM | 624 | CG1 | ILE | 42 | 11.145 | -0.836 | -0.860 | 1.00 | 0.00 |
| ATOM | 625 | HG11 | ILE | 42 | 11.496 | 0.180 | -0.962 | 1.00 | 0.00 |
| ATOM | 626 | HG12 | ILE | 42 | 10.777 | -1.186 | -1.813 | 1.00 | 0.00 |
| ATOM | 627 | CG2 | ILE | 42 | 9.443 | -2.298 | 0.224 | 1.00 | 0.00 |
| ATOM | 628 | HG21 | ILE | 42 | 9.306 | -2.663 | -0.783 | 1.00 | 0.00 |
| ATOM | 629 | HG22 | ILE | 42 | 8.494 | -2.287 | 0.737 | 1.00 | 0.00 |
| ATOM | 630 | HG23 | ILE | 42 | 10.131 | -2.941 | 0.750 | 1.00 | 0.00 |
| ATOM | 631 | CD1 | ILE | 42 | 12.301 | -1.729 | -0.400 | 1.00 | 0.00 |
| ATOM | 632 | HD11 | ILE | 42 | 12.203 | -2.705 | -0.854 | 1.00 | 0.00 |
| ATOM | 633 | HD12 | ILE | 42 | 12.275 | -1.825 | 0.675 | 1.00 | 0.00 |
| ATOM | 634 | HD13 | ILE | 42 | 13.239 | -1.285 | -0.700 | 1.00 | 0.00 |
| ATOM | 635 | C | ILE | 42 | 7.888 | 0.233 | 0.907 | 1.00 | 0.00 |
| ATOM | 636 | O | ILE | 42 | 8.249 | 0.470 | 2.044 | 1.00 | 0.00 |
| ATOM | 637 | N | CYS | 43 | 6.621 | 0.082 | 0.604 | 1.00 | 0.00 |
| ATOM | 638 | HN | CYS | 43 | 6.363 | -0.113 | -0.322 | 1.00 | 0.00 |
| ATOM | 639 | CA | CYS | 43 | 5.569 | 0.198 | 1.656 | 1.00 | 0.00 |
| ATOM | 640 | HA | CYS | 43 | 5.746 | 1.082 | 2.251 | 1.00 | 0.00 |
| ATOM | 641 | HB1 | CYS | 43 | 3.453 | -0.182 | 1.629 | 1.00 | 0.00 |
| ATOM | 642 | HB2 | CYS | 43 | 4.214 | -0.215 | 0.039 | 1.00 | 0.00 |
| ATOM | 643 | C | CYS | 43 | 5.621 | -1.042 | 2.550 | 1.00 | 0.00 |
| ATOM | 644 | O | CYS | 43 | 5.914 | -2.131 | 2.093 | 1.00 | 0.00 |
| ATOM | 645 | CB | CYS | 43 | 4.189 | 0.289 | 0.993 | 1.00 | 0.00 |
| ATOM | 646 | SG | CYS | 43 | 3.742 | 2.028 | 0.741 | 1.00 | 0.00 |
| ATOM | 647 | N | SER | 44 | 5.342 | -0.887 | 3.820 | 1.00 | 0.00 |
| ATOM | 648 | HN | SER | 44 | 5.111 | 0.002 | 4.163 | 1.00 | 0.00 |
| ATOM | 649 | CA | SER | 44 | 5.377 | -2.057 | 4.746 | 1.00 | 0.00 |
| ATOM | 650 | HA | SER | 44 | 5.988 | -2.837 | 4.317 | 1.00 | 0.00 |
| ATOM | 651 | CB | SER | 44 | 5.970 | -1.626 | 6.089 | 1.00 | 0.00 |
| ATOM | 652 | HB1 | SER | 44 | 7.044 | -1.758 | 6.065 | 1.00 | 0.00 |
| ATOM | 653 | HB2 | SER | 44 | 5.555 | -2.229 | 6.880 | 1.00 | 0.00 |
| ATOM | 654 | OG | SER | 44 | 5.652 | -0.261 | 6.327 | 1.00 | 0.00 |
| ATOM | 655 | HG | SER | 44 | 6.453 | 0.254 | 6.213 | 1.00 | 0.00 |
| ATOM | 656 | C | SER | 44 | 3.958 | -2.582 | 4.966 | 1.00 | 0.00 |
| ATOM | 657 | O | SER | 44 | 2.988 | -1.872 | 4.776 | 1.00 | 0.00 |
| ATOM | 658 | N | LEU | 45 | 3.831 | -3.821 | 5.374 | 1.00 | 0.00 |
| ATOM | 659 | HN | LEU | 45 | 4.633 | -4.366 | 5.523 | 1.00 | 0.00 |
| ATOM | 660 | CA | LEU | 45 | 2.479 | -4.408 | 5.619 | 1.00 | 0.00 |
| ATOM | 661 | HA | LEU | 45 | 1.902 | -4.381 | 4.707 | 1.00 | 0.00 |
| ATOM | 662 | CB | LEU | 45 | 2.642 | -5.866 | 6.086 | 1.00 | 0.00 |
| ATOM | 663 | HB1 | LEU | 45 | 2.210 | -5.981 | 7.070 | 1.00 | 0.00 |
| ATOM | 664 | HB2 | LEU | 45 | 3.693 | -6.109 | 6.130 | 1.00 | 0.00 |
| ATOM | 665 | CG | LEU | 45 | 1.940 | -6.828 | 5.113 | 1.00 | 0.00 |
| ATOM | 666 | HG | LEU | 45 | 2.092 | -7.842 | 5.453 | 1.00 | 0.00 |
| ATOM | 667 | CD1 | LEU | 45 | 0.437 | -6.533 | 5.085 | 1.00 | 0.00 |
| ATOM | 668 | HD11 | LEU | 45 | 0.254 | -5.543 | 5.475 | 1.00 | 0.00 |
| ATOM | 669 | HD12 | LEU | 45 | -0.083 | -7.259 | 5.692 | 1.00 | 0.00 |
| ATOM | 670 | HD13 | LEU | 45 | 0.074 | -6.591 | 4.068 | 1.00 | 0.00 |
| ATOM | 671 | CD2 | LEU | 45 | 2.530 | -6.674 | 3.700 | 1.00 | 0.00 |
| ATOM | 672 | HD21 | LEU | 45 | 1.731 | -6.619 | 2.977 | 1.00 | 0.00 |
| ATOM | 673 | HD22 | LEU | 45 | 3.154 | -7.528 | 3.478 | 1.00 | 0.00 |
| ATOM | 674 | HD23 | LEU | 45 | 3.126 | -5.774 | 3.650 | 1.00 | 0.00 |
| ATOM | 675 | C | LEU | 45 | 1.763 | -3.590 | 6.700 | 1.00 | 0.00 |
| ATOM | 676 | O | LEU | 45 | 0.555 | -3.464 | 6.698 | 1.00 | 0.00 |
| ATOM | 677 | N | TYR | 46 | 2.512 | -3.032 | 7.616 | 1.00 | 0.00 |
| ATOM | 678 | HN | TYR | 46 | 3.486 | -3.149 | 7.586 | 1.00 | 0.00 |
| ATOM | 679 | CA | TYR | 46 | 1.903 | -2.213 | 8.702 | 1.00 | 0.00 |
| ATOM | 680 | HA | TYR | 46 | 1.032 | -2.718 | 9.092 | 1.00 | 0.00 |
| ATOM | 681 | CB | TYR | 46 | 2.945 | -2.040 | 9.826 | 1.00 | 0.00 |
| ATOM | 682 | HB1 | TYR | 46 | 3.877 | -1.704 | 9.396 | 1.00 | 0.00 |
| ATOM | 683 | HB2 | TYR | 46 | 3.100 | -2.989 | 10.316 | 1.00 | 0.00 |
| ATOM | 684 | CG | TYR | 46 | 2.474 | -1.024 | 10.846 | 1.00 | 0.00 |
| ATOM | 685 | CD1 | TYR | 46 | 1.239 | -1.187 | 11.485 | 1.00 | 0.00 |
| ATOM | 686 | HD1 | TYR | 46 | 0.620 | -2.044 | 11.256 | 1.00 | 0.00 |
| ATOM | 687 | CD2 | TYR | 46 | 3.274 | 0.084 | 11.139 | 1.00 | 0.00 |
| ATOM | 688 | HD2 | TYR | 46 | 4.228 | 0.205 | 10.644 | 1.00 | 0.00 |
| ATOM | 689 | CE1 | TYR | 46 | 0.806 | -0.239 | 12.419 | 1.00 | 0.00 |
| ATOM | 690 | HE1 | TYR | 46 | -0.147 | -0.362 | 12.913 | 1.00 | 0.00 |
| ATOM | 691 | CE2 | TYR | 46 | 2.843 | 1.031 | 12.074 | 1.00 | 0.00 |
| ATOM | 692 | HE2 | TYR | 46 | 3.462 | 1.886 | 12.298 | 1.00 | 0.00 |
| ATOM | 693 | CZ | TYR | 46 | 1.608 | 0.871 | 12.714 | 1.00 | 0.00 |
| ATOM | 694 | OH | TYR | 46 | 1.182 | 1.807 | 13.635 | 1.00 | 0.00 |
| ATOM | 695 | HH | TYR | 46 | 1.440 | 2.676 | 13.317 | 1.00 | 0.00 |
| ATOM | 696 | C | TYR | 46 | 1.489 | -0.848 | 8.130 | 1.00 | 0.00 |
| ATOM | 697 | O | TYR | 46 | 0.498 | -0.273 | 8.537 | 1.00 | 0.00 |
| ATOM | 698 | N | GLN | 47 | 2.250 | -0.329 | 7.202 | 1.00 | 0.00 |
| ATOM | 699 | HN | GLN | 47 | 3.049 | -0.812 | 6.900 | 1.00 | 0.00 |
| ATOM | 700 | CA | GLN | 47 | 1.917 | 0.999 | 6.609 | 1.00 | 0.00 |
| ATOM | 701 | HA | GLN | 47 | 1.816 | 1.727 | 7.398 | 1.00 | 0.00 |
| ATOM | 702 | CB | GLN | 47 | 3.037 | 1.432 | 5.663 | 1.00 | 0.00 |
| ATOM | 703 | HB1 | GLN | 47 | 2.649 | 2.158 | 4.965 | 1.00 | 0.00 |
| ATOM | 704 | HB2 | GLN | 47 | 3.403 | 0.573 | 5.123 | 1.00 | 0.00 |
| ATOM | 705 | CG | GLN | 47 | 4.180 | 2.060 | 6.463 | 1.00 | 0.00 |
| ATOM | 706 | HG1 | GLN | 47 | 5.107 | 1.930 | 5.927 | 1.00 | 0.00 |
| ATOM | 707 | HG2 | GLN | 47 | 4.251 | 1.582 | 7.429 | 1.00 | 0.00 |
| ATOM | 708 | CD | GLN | 47 | 3.908 | 3.555 | 6.649 | 1.00 | 0.00 |
| ATOM | 709 | OE1 | GLN | 47 | 2.774 | 3.961 | 6.811 | 1.00 | 0.00 |
| ATOM | 710 | NE2 | GLN | 47 | 4.907 | 4.396 | 6.632 | 1.00 | 0.00 |
| ATOM | 711 | HE21 | GLN | 47 | 5.821 | 4.070 | 6.501 | 1.00 | 0.00 |
| ATOM | 712 | HE22 | GLN | 47 | 4.740 | 5.355 | 6.751 | 1.00 | 0.00 |
| ATOM | 713 | C | GLN | 47 | 0.606 | 0.914 | 5.822 | 1.00 | 0.00 |
| ATOM | 714 | O | GLN | 47 | -0.167 | 1.855 | 5.797 | 1.00 | 0.00 |
| ATOM | 715 | N | LEU | 48 | 0.356 | -0.193 | 5.164 | 1.00 | 0.00 |
| ATOM | 716 | HN | LEU | 48 | 0.999 | -0.932 | 5.186 | 1.00 | 0.00 |
| ATOM | 717 | CA | LEU | 48 | -0.898 | -0.317 | 4.361 | 1.00 | 0.00 |
| ATOM | 718 | HA | LEU | 48 | -1.178 | 0.661 | 4.011 | 1.00 | 0.00 |
| ATOM | 719 | CB | LEU | 48 | -0.653 | -1.227 | 3.151 | 1.00 | 0.00 |
| ATOM | 720 | HB1 | LEU | 48 | -1.599 | -1.466 | 2.688 | 1.00 | 0.00 |
| ATOM | 721 | HB2 | LEU | 48 | -0.178 | -2.139 | 3.482 | 1.00 | 0.00 |
| ATOM | 722 | CG | LEU | 48 | 0.250 | -0.524 | 2.127 | 1.00 | 0.00 |
| ATOM | 723 | HG | LEU | 48 | 1.213 | -0.323 | 2.575 | 1.00 | 0.00 |
| ATOM | 724 | CD1 | LEU | 48 | 0.434 | -1.434 | 0.914 | 1.00 | 0.00 |
| ATOM | 725 | HD11 | LEU | 48 | -0.381 | -1.278 | 0.222 | 1.00 | 0.00 |
| ATOM | 726 | HD12 | LEU | 48 | 0.441 | -2.465 | 1.236 | 1.00 | 0.00 |
| ATOM | 727 | HD13 | LEU | 48 | 1.369 | -1.202 | 0.427 | 1.00 | 0.00 |
| ATOM | 728 | CD2 | LEU | 48 | -0.391 | 0.793 | 1.667 | 1.00 | 0.00 |
| ATOM | 729 | HD21 | LEU | 48 | -0.293 | 1.532 | 2.447 | 1.00 | 0.00 |
| ATOM | 730 | HD22 | LEU | 48 | -1.437 | 0.629 | 1.455 | 1.00 | 0.00 |
| ATOM | 731 | HD23 | LEU | 48 | 0.108 | 1.143 | 0.775 | 1.00 | 0.00 |
| ATOM | 732 | C | LEU | 48 | -2.047 | -0.892 | 5.201 | 1.00 | 0.00 |
| ATOM | 733 | O | LEU | 48 | -3.135 | -1.097 | 4.695 | 1.00 | 0.00 |
| ATOM | 734 | N | GLU | 49 | -1.832 | -1.150 | 6.468 | 1.00 | 0.00 |
| ATOM | 735 | HN | GLU | 49 | -0.954 | -0.979 | 6.864 | 1.00 | 0.00 |
| ATOM | 736 | CA | GLU | 49 | -2.930 | -1.700 | 7.315 | 1.00 | 0.00 |
| ATOM | 737 | HA | GLU | 49 | -3.371 | -2.553 | 6.822 | 1.00 | 0.00 |
| ATOM | 738 | CB | GLU | 49 | -2.361 | -2.134 | 8.669 | 1.00 | 0.00 |
| ATOM | 739 | HB1 | GLU | 49 | -3.148 | -2.130 | 9.407 | 1.00 | 0.00 |
| ATOM | 740 | HB2 | GLU | 49 | -1.583 | -1.445 | 8.968 | 1.00 | 0.00 |
| ATOM | 741 | CG | GLU | 49 | -1.773 | -3.547 | 8.552 | 1.00 | 0.00 |
| ATOM | 742 | HG1 | GLU | 49 | -0.698 | -3.485 | 8.536 | 1.00 | 0.00 |
| ATOM | 743 | HG2 | GLU | 49 | -2.119 | -4.009 | 7.640 | 1.00 | 0.00 |
| ATOM | 744 | CD | GLU | 49 | -2.211 | -4.392 | 9.751 | 1.00 | 0.00 |
| ATOM | 745 | OE1 | GLU | 49 | -2.509 | -5.558 | 9.550 | 1.00 | 0.00 |
| ATOM | 746 | OE2 | GLU | 49 | -2.237 | -3.860 | 10.848 | 1.00 | 0.00 |
| ATOM | 747 | C | GLU | 49 | -4.006 | -0.631 | 7.530 | 1.00 | 0.00 |
| ATOM | 748 | O | GLU | 49 | -5.146 | -0.942 | 7.823 | 1.00 | 0.00 |
| ATOM | 749 | N | ASN | 50 | -3.655 | 0.624 | 7.393 | 1.00 | 0.00 |
| ATOM | 750 | HN | ASN | 50 | -2.730 | 0.851 | 7.163 | 1.00 | 0.00 |
| ATOM | 751 | CA | ASN | 50 | -4.655 | 1.714 | 7.596 | 1.00 | 0.00 |
| ATOM | 752 | HA | ASN | 50 | -5.281 | 1.470 | 8.441 | 1.00 | 0.00 |
| ATOM | 753 | CB | ASN | 50 | -3.919 | 3.024 | 7.881 | 1.00 | 0.00 |
| ATOM | 754 | HB1 | ASN | 50 | -4.525 | 3.856 | 7.552 | 1.00 | 0.00 |
| ATOM | 755 | HB2 | ASN | 50 | -2.979 | 3.032 | 7.348 | 1.00 | 0.00 |
| ATOM | 756 | CG | ASN | 50 | -3.657 | 3.151 | 9.383 | 1.00 | 0.00 |
| ATOM | 757 | OD1 | ASN | 50 | -4.068 | 4.112 | 10.004 | 1.00 | 0.00 |
| ATOM | 758 | ND2 | ASN | 50 | -2.983 | 2.218 | 9.997 | 1.00 | 0.00 |
| ATOM | 759 | HD21 | ASN | 50 | -2.650 | 1.444 | 9.497 | 1.00 | 0.00 |
| ATOM | 760 | HD22 | ASN | 50 | -2.810 | 2.291 | 10.959 | 1.00 | 0.00 |
| ATOM | 761 | C | ASN | 50 | -5.538 | 1.894 | 6.347 | 1.00 | 0.00 |
| ATOM | 762 | O | ASN | 50 | -6.396 | 2.757 | 6.321 | 1.00 | 0.00 |
| ATOM | 763 | N | TYR | 51 | -5.342 | 1.102 | 5.313 | 1.00 | 0.00 |
| ATOM | 764 | HN | TYR | 51 | -4.649 | 0.413 | 5.342 | 1.00 | 0.00 |
| ATOM | 765 | CA | TYR | 51 | -6.184 | 1.260 | 4.086 | 1.00 | 0.00 |
| ATOM | 766 | HA | TYR | 51 | -6.744 | 2.180 | 4.159 | 1.00 | 0.00 |
| ATOM | 767 | CB | TYR | 51 | -5.293 | 1.305 | 2.838 | 1.00 | 0.00 |
| ATOM | 768 | HB1 | TYR | 51 | -5.911 | 1.234 | 1.956 | 1.00 | 0.00 |
| ATOM | 769 | HB2 | TYR | 51 | -4.601 | 0.476 | 2.860 | 1.00 | 0.00 |
| ATOM | 770 | CG | TYR | 51 | -4.525 | 2.597 | 2.799 | 1.00 | 0.00 |
| ATOM | 771 | CD1 | TYR | 51 | -5.154 | 3.766 | 2.364 | 1.00 | 0.00 |
| ATOM | 772 | HD1 | TYR | 51 | -6.189 | 3.738 | 2.061 | 1.00 | 0.00 |
| ATOM | 773 | CD2 | TYR | 51 | -3.187 | 2.625 | 3.189 | 1.00 | 0.00 |
| ATOM | 774 | HD2 | TYR | 51 | -2.706 | 1.719 | 3.518 | 1.00 | 0.00 |
| ATOM | 775 | CE1 | TYR | 51 | -4.442 | 4.970 | 2.323 | 1.00 | 0.00 |
| ATOM | 776 | HE1 | TYR | 51 | -4.924 | 5.873 | 1.980 | 1.00 | 0.00 |
| ATOM | 777 | CE2 | TYR | 51 | -2.470 | 3.824 | 3.147 | 1.00 | 0.00 |
| ATOM | 778 | HE2 | TYR | 51 | -1.435 | 3.843 | 3.448 | 1.00 | 0.00 |
| ATOM | 779 | CZ | TYR | 51 | -3.098 | 4.998 | 2.716 | 1.00 | 0.00 |
| ATOM | 780 | OH | TYR | 51 | -2.392 | 6.183 | 2.678 | 1.00 | 0.00 |
| ATOM | 781 | HH | TYR | 51 | -2.342 | 6.527 | 3.574 | 1.00 | 0.00 |
| ATOM | 782 | C | TYR | 51 | -7.163 | 0.085 | 3.961 | 1.00 | 0.00 |
| ATOM | 783 | O | TYR | 51 | -7.191 | -0.606 | 2.958 | 1.00 | 0.00 |
| ATOM | 784 | N | CYS | 52 | -7.969 | -0.146 | 4.964 | 1.00 | 0.00 |
| ATOM | 785 | HN | CYS | 52 | -7.933 | 0.419 | 5.764 | 1.00 | 0.00 |
| ATOM | 786 | CA | CYS | 52 | -8.945 | -1.273 | 4.885 | 1.00 | 0.00 |
| ATOM | 787 | HA | CYS | 52 | -9.067 | -1.561 | 3.852 | 1.00 | 0.00 |
| ATOM | 788 | HB1 | CYS | 52 | -9.205 | -3.211 | 5.770 | 1.00 | 0.00 |
| ATOM | 789 | HB2 | CYS | 52 | -8.165 | -2.127 | 6.692 | 1.00 | 0.00 |
| ATOM | 790 | C | CYS | 52 | -10.299 | -0.835 | 5.445 | 1.00 | 0.00 |
| ATOM | 791 | O | CYS | 52 | -10.402 | 0.139 | 6.168 | 1.00 | 0.00 |
| ATOM | 792 | CB | CYS | 52 | -8.430 | -2.462 | 5.700 | 1.00 | 0.00 |
| ATOM | 793 | SG | CYS | 52 | -6.973 | -3.180 | 4.902 | 1.00 | 0.00 |
| ATOM | 794 | N | ASN | 53 | -11.337 | -1.559 | 5.114 | 1.00 | 0.00 |
| ATOM | 795 | HN | ASN | 53 | -11.218 | -2.339 | 4.531 | 1.00 | 0.00 |
| ATOM | 796 | CA | ASN | 53 | -12.699 | -1.212 | 5.617 | 1.00 | 0.00 |
| ATOM | 797 | HA | ASN | 53 | -12.919 | -0.183 | 5.382 | 1.00 | 0.00 |
| ATOM | 798 | CB | ASN | 53 | -13.733 | -2.121 | 4.950 | 1.00 | 0.00 |
| ATOM | 799 | HB1 | ASN | 53 | -14.565 | -2.272 | 5.621 | 1.00 | 0.00 |
| ATOM | 800 | HB2 | ASN | 53 | -13.279 | -3.074 | 4.721 | 1.00 | 0.00 |
| ATOM | 801 | CG | ASN | 53 | -14.234 | -1.471 | 3.658 | 1.00 | 0.00 |
| ATOM | 802 | OD1 | ASN | 53 | -13.551 | -0.657 | 3.069 | 1.00 | 0.00 |
| ATOM | 803 | ND2 | ASN | 53 | -15.407 | -1.800 | 3.192 | 1.00 | 0.00 |
| ATOM | 804 | HD21 | ASN | 53 | -15.958 | -2.456 | 3.669 | 1.00 | 0.00 |
| ATOM | 805 | HD22 | ASN | 53 | -15.737 | -1.391 | 2.364 | 1.00 | 0.00 |
| ATOM | 806 | C | ASN | 53 | -12.750 | -1.410 | 7.133 | 1.00 | 0.00 |
| ATOM | 807 | OT1 | ASN | 53 | -12.763 | -2.553 | 7.560 | 1.00 | 0.00 |
| ATOM | 808 | OT2 | ASN | 53 | -12.775 | -0.416 | 7.840 | 1.00 | 0.00 |
| END | | | | | | | | | |

### Example 3

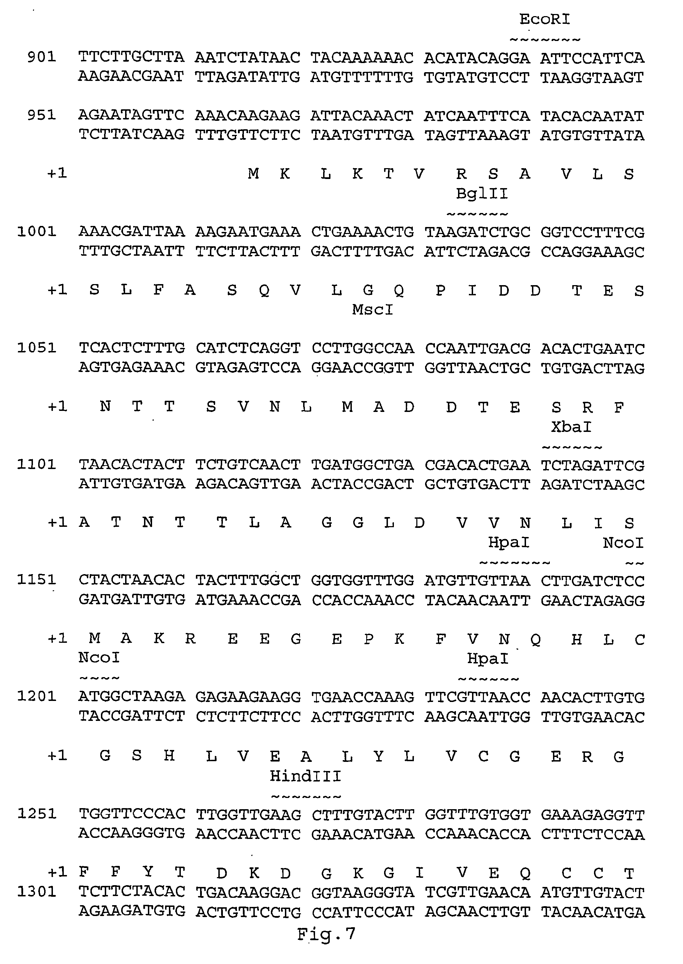

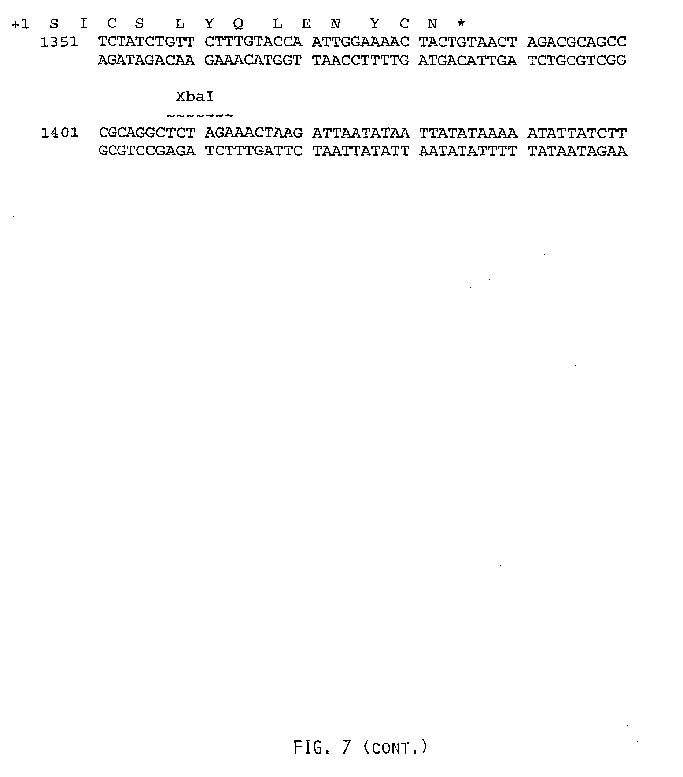

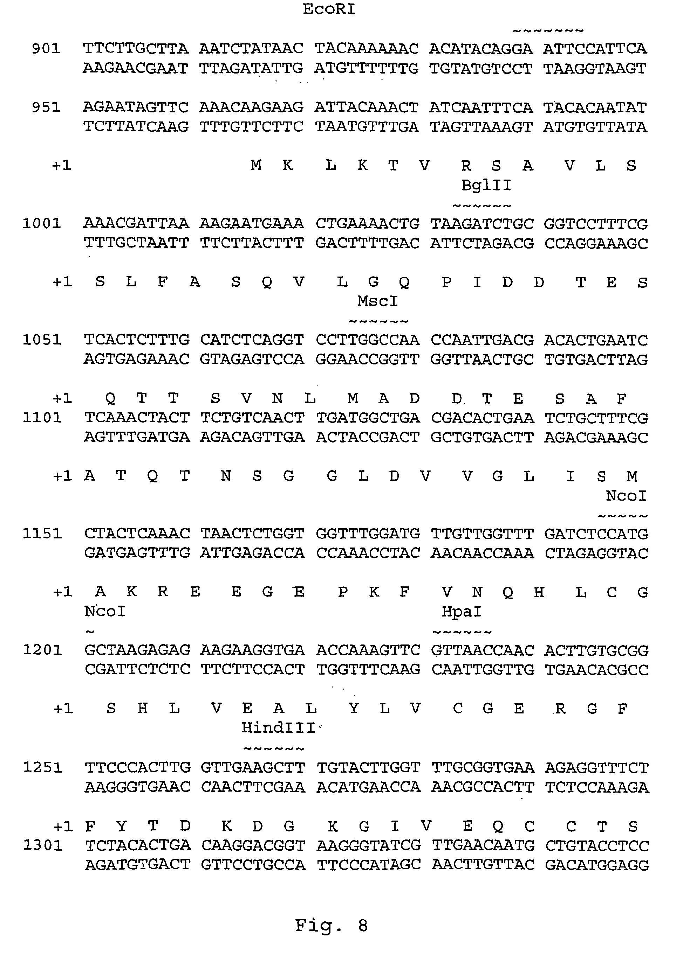

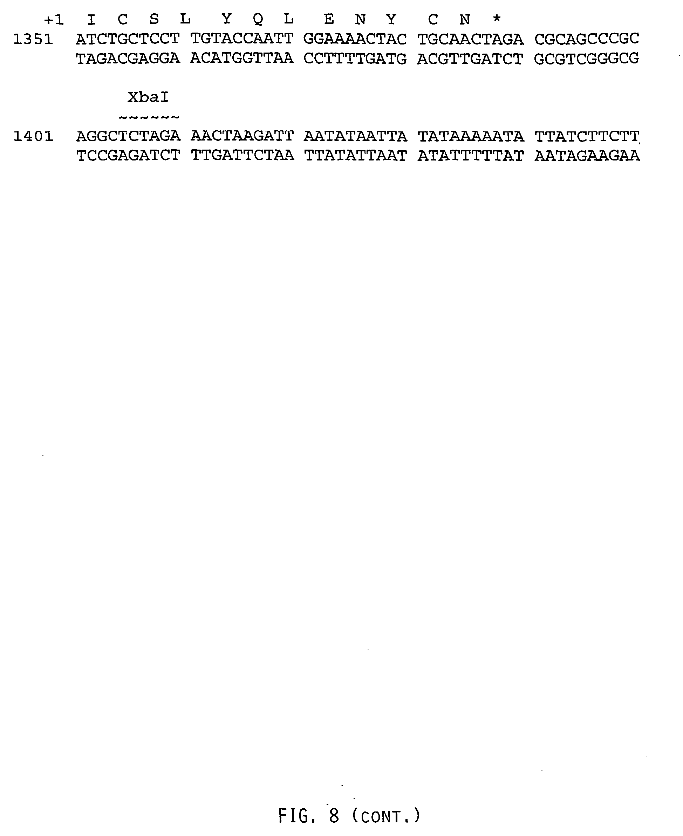

The insulin analogue precursor Asp^{B28}IP(AspGlyLys) was produced culturing yeast strain MT663 transformed with an expression plasmid expressing either a YAP3-TA39-EEGEPK(SEQ ID NO:8)-Asp^{B28}IP(DGK) fusion protein or a YAP3-TA57-EEGEPK(SEQ ID NO:8)-Asp^{B28}IP(DGK) fusion protein. TA39 is a pro-sequence QPIDDTESNTTSVNLMADDT-ESRFATNTTLAGGLDWNLISMAKR(SEQ ID NO:15). The sequence EEGEPK(SEQ ID NO:8) is an N-terminal extension to the B-chain of the insulin analogue. TA57 is a pro-sequence QPIDDTESQTTSVNLMADDTESAFATQTNSGGLDWGLISMAKR (SEQ ID NO:16). cDNA encoding the leader sequences YAP3-TA39 and YAP3-TA57 and cDNA encoding the Asp^{B28}IP(DGK) and the N-terminal extension were cloned into an expression vector of the C-POT type using standard techniques (Sambrook J, Fritsch EF and Maniatis T, Molecular cloning, Cold spring Harbour laboratory press, 1989). The DNA and inferred amino acids sequences are shown in Fig 7(SEQ ID NO:9 and 10) and Fig 8 (SEQ ID NO:11 and 12).

Table 5 shows the yields. Fermentation was conducted at 30°C for 72 h in 5 ml YPD. IP yield was determined by RP-HPLC of the culture supernatant and is expressed relative to the IP yield of the strain yJB155.

**TABLE 5**

| Leader | Precursor | N-terminal extension | C-peptide | Yield* | SEQ ID |
|---|---|---|---|---|---|
| α*-ex4 | Asp^{B28}IP | | None | 100 | SEQ ID NO:3 |
| YAP3-TA39 | Asp^{B28}IP | GluGluGlyGluProLys | AspGlyLys | 477% | SEQ ID NO:8 |
| YAP3-TA57 | Asp^{B28}IP | GluGluGlyGluProLys | AspGlyLys | 306% | SEQ ID NO:8 |

## Claims

1. An insulin precursor or insulin precursor analogue comprising a sequence of formula I:
B(1-27) - X₃ - X₂ - X₁ - Y - A(1-21),
wherein
A(1-21) are the amino acids 1 to 21 of the A chain of human insulin,
B(1-27) are the amino acids 1 to 27 of the B chain of human insulin,
X₁ is 1-3 amino acid residues in length and comprises a single Gly immediately N-terminal to Y,
X₂ is one of Pro, Lys, Ala, Arg or Pro-Thr at position 29 of the B chain,
X₃ is one of Pro, Asp, Lys, or Ile at position 28 of the B chain, and
Y is Lys or Arg.

2. An insulin precursor or an insulin precursor analogue according to claim 1, wherein X₃ is Asp and X₂ is Lys.

3. An insulin precursor or an insulin precursor analogue according to claim 1, wherein X₃ is Pro and X₂ is Lys.

4. An insulin precursor or an insulin precursor analogue according to any of claims 1-3 wherein X₁ - Y is selected from the group of:(a) Glu-Glu-Gly-Lys(SEQ ID NO:1) (b) Glu-Gly-Lys, (c) Ser-Gly-Lys, (d) Asn-Gly-Lys, (e) Thr-Gly-Lys, (f) Asp-Gly-Lys, (g) Me-Gly-Lys, (h) Ala-Gly-Lys, (i) His-Gly-Lys and (j) Gly-Lys.

5. A polynucleotide sequence encoding an insulin precursor or an insulin precursor analogue according to any of claims 1-4.

6. An expression vector comprising a polynucleotide sequence according to claim 5.

7. A host cell transformed with the vector according to claim 6.

8. A process for making an insulin precursor or an insulin precursor analogue said method comprising (i) culturing a host cell comprising a polynucleotide sequence encoding an insulin precursor or an insulin precursor analogue according to any of claims 1-4 under suitable culture conditions for expression of said precursor or precursor analogue; and (ii) isolating the expressed precursor or precursor analogue.

9. A process according to claim 8, wherein the host cell is a yeast host cell.

10. A process for making insulin or an insulin analogue, said method comprising (i) culturing a host cell comprising a polynucleotide sequence encoding the insulin precursor or an insulin precursor analogue according to any of claims 1-4 under suitable culture conditions for expression of said precursor or precursor analogue; (ii) isolating the precursor or precursor analogue from the culture medium and (iii) converting the precursor or precursor analogue into insulin or an insulin analogue by in vitro chemical or enzymatic conversion.

11. A process according to claim 10, wherein the host cell is a yeast host cell line.

## Patentansprüche

1. Insulinvorläufer oder Insulinvorläuferanalogon, umfassend eine Sequenz der Formel I:
B(1-27) - X₃ - X₂ - X₁ - Y - A(1-21)
wobei
A(1-21) die Aminosäuren 1 bis 21 der A-Kette von Humaninsulin sind,
B(1-27) die Aminosäuren 1 bis 27 der B-Kette von Humaninsulin sind,
X₁ 1-3 Aminosäurereste lang ist und unmittelbar N-terminal zu Y ein einzelnes Gly umfasst,
X₂ eines von Pro, Lys, Ala, Arg oder Pro-Thr an Position 29 der B-Kette ist,
X₃ eines von Pro, Asp, Lys oder Ile an Position 28 der B-Kette ist und
Y Lys oder Arg ist.

2. Insulinvorläufer oder Insulinvorläuferanalogon nach Anspruch 1, wobei X₃ Asp ist und X₂ Lys ist.

3. Insulinvorläufer oder Insulinvorläuferanalogon nach Anspruch 1, wobei X₃ Pro ist und X₂ Lys ist.

4. Insulinvorläufer oder Insulinvorläuferanalogon nach einem der Ansprüche 1-3, wobei X₁ - Y ausgewählt ist aus der Gruppe: (a) Glu-Glu-Gly-Lys(SEQ ID NR: 1), (b) Glu-Gly-Lys, (c) Ser-Gly-Lys, (d) Asn-Gly-Lys, (e) Thr-Gly-Lys, (f) Asp-Gly-Lys, (g) Me-Gly-Lys, (h) Ala-Gly-Lys, (i) His-Gly-Lys und (j) Gly-Lys.

5. Polynukleotidsequenz, die einen Insulinvorläufer oder ein Insulinvorläuferanalogon nach einem der Ansprüche 1-4 kodiert.

6. Expressionsvektor, der eine Polynukleotidsequenz nach Anspruch 5 umfasst.

7. Wirtszelle, die mit dem Vektor nach Anspruch 6 transformiert ist.

8. Verfahren zur Herstellung eines Insulinvorläufers oder Insulinvorläuferanalogons, wobei das Verfahren (i) das Kultivieren einer Wirtszelle, die eine Polynukleotidsequenz umfasst, die einen Insulinvorläufer oder ein Insulinvorläuferanalogon nach einem der Ansprüche 1-4 kodiert, unter geeigneten Kulturbedingungen zur Expression des Vorläufers oder Vorläuferanalogons; und (ii) Isolieren des exprimierten Vorläufers oder Vorläuferanalogons umfasst.

9. Verfahren nach Anspruch 8, wobei die Wirtszelle eine Hefewirtszelle ist.

10. Verfahren zur Herstellung von Insulin oder eines Insulinanalogons, wobei das Verfahren (i) das Kultivieren einer Wirtszelle, die eine Polynukleotidsequenz umfasst, die einen Insulinvorläufer oder ein Insulinvorläuferanalogon nach einem der Ansprüche 1-4 kodiert, unter geeigneten Kulturbedingungen zur Expression des Vorläufers oder Vorläuferanalogons; (ii) Isolieren des Vorläufers oder Vorläuferanalogons aus dem Kulturmedium und (iii) Umwandeln des Vorläufers oder Vorläuferanalogons in Insulin oder ein Insulinanalogon durch chemische oder enzymatische Umwandlung in vitro umfasst.

11. Verfahren nach Anspruch 10, wobei die Wirtszelle eine Hefewirtszelllinie ist.

## Revendications

1. Précurseur de l'insuline ou analogue d'un précurseur de l'insuline, comprenant une séquence de formule I :
B(1-27) - X₃ - X₂ - X₁ - Y - A(1-21),
dans laquelle
A(1-21) correspond aux acides aminés 1 à 21 de la chaîne A de l'insuline humaine,
B(1-27) correspond aux acides aminés 1 à 27 de la chaîne B de l'insuline humaine,
X₁ a une longueur de 1-3 résidus d'acides aminés et comprend un Gly unique en position immédiatement N-terminale par rapport à Y,
X₂ est l'un de Pro, Lys, Ala, Arg ou Pro-Thr sur la position 29 de la chaîne B,
X₃ est l'un de Pro, Asp, Lys ou Ile sur la position 28 de la chaîne B, et
Y est Lys ou Arg.

2. Précurseur de l'insuline ou analogue d'un précurseur de l'insuline selon la revendication 1, dans lequel X₃ est Asp et X₂ es Lys.

3. Précurseur de l'insuline ou analogue d'un précurseur de l'insuline selon la revendication 1, dans lequel X₃ est Pro et X₂ est Lys.

4. Précurseur de l'insuline ou analogue d'un précurseur de l'insuline selon l'une quelconque des revendications 1-3, dans lequel X₁-Y est choisi dans le groupe consistant sen : (a) Glu-Glu-Gly-Lys(SEQ ID N°1), (b) Glu-Gly-Lys, (c) Ser-Gly-Lys, (d) Asn-Gly-Lys, (e) Thr-Gly-Lys, (f) Asp-Gly-Lys, (g) Me-Gly-Lys, (h) Ala-Gly-Lys, (i) His-Gly-Lys et (j) Gly-Lys.

5. Séquence polynucléotidique codant pour un précurseur de l'insuline ou un analogue d'un précurseur de l'insuline selon l'une quelconque des revendications 1 à 4.

6. Vecteur d'expression comprenant une séquence polynucléotidique selon la revendication 5.

7. Cellule hôte transformée avec le vecteur selon la revendication 6.

8. Procédé de préparation d'un précurseur de l'insuline ou d'un analogue d'un précurseur de l'insuline, ledit procédé comprenant (i) la culture d'une cellule hôte comprenant une séquence polynucléotidique codant pour un précurseur de l'insuline ou un analogue d'un précurseur de l'insuline selon l'une quelconque des revendications 1-4 dans des conditions de culture convenant à l'expression dudit précurseur ou dudit analogue de précurseur ; et (ii) l'isolement du précurseur ou de l'analogue de précurseur exprimé.

9. Procédé selon la revendication 8, dans lequel la cellule hôte est une cellule hôte de levure.

10. Procédé de préparation d'insuline ou d'un analogue d'insuline, ledit procédé comprenant (i) la culture d'une cellule hôte comprenant une séquence polynucléotidique codant pour le précurseur d'insuline ou un analogue de précurseur d'insuline selon l'une quelconque des revendications 1-4 dans des conditions de culture convenant à l'expression dudit précurseur ou analogue de précurseur ; (ii) isolement, d'avec le milieu de culture, du précurseur ou de l'analogue de précurseur ; et (iii) conversion du précurseur ou de l'analogue de précurseur en insuline ou en un analogue de l'insuline par conversion chimique ou enzymatique in vitro.

11. Procédé selon la revendication 10, dans lequel la cellule hôte est une lignée de cellules hôtes de levure.


### SEQUENCE LISTING

<110> Novo Nordisk A/S
   Kjeldsen, Thomas
   Ludvigsen, Svend
<120> Method for making insulin precursors and insulin precursor an alogs
<130> 6148.204-WO
<150> DK PA 1999 011869
   <151> 1999-12-29
<160> 16
<170> PatentIn version 3.0
<210> 1
   <211> 4
   <212> PRT
   <213> Mini C-peptide
<400> 1
   Glu Glu Gly Lys
   1
<210> 2
   <211> 10
   <212> PRT
   <213> N-terminal extension
<400> 2
   Glu Glu Ala Glu Ala Glu Ala Glu Pro Lys
   1               5                   10
<210> 3
   <211> 9
   <212> PRT
   <213> N-terminal
<400> 3
   Glu Glu Ala Glu Ala Glu Ala Pro Lys
   1               5
<210> 4
   <211> 600
   <212> DNA
   <213> Alpha leader fused with N-terminally extended AspB28 IP
<220>
   <221> CDS
   <222> (114)..(545)
<400> 4
   tcttgcttaa atctataact acaaaaaaca catacaggaa ttccattcaa gatctgttca
     60
   aacaagaaga ttacaaacta tcaatttcat acacaatata aacgattaaa aga atg
    116
                                                              Met
                                                              1
   aga ttt cct tca att ttt act gca gtt tta ttc gca gca tcc tcc gca
    164
   Arg Phe Pro Ser Ile Phe Thr Ala Val Leu Phe Ala Ala Ser Ser Ala
                   5                   10                  15
   tta gct gct cca gtc aac act aca aca gaa gat gaa acg gca caa att
    212
   Leu Ala Ala Pro Val Asn Thr Thr Thr Glu Asp Glu Thr Ala Gln Ile
               20                  25                  30
   ccg gct gaa gct gtc atc ggt tac tca gat tta gaa ggg gat ttc gat
    260
   Pro Ala Glu Ala Val Ile Gly Tyr Ser Asp Leu Glu Gly Asp Phe Asp
           35                  40                  45
   gtt gct gtt ttg cca ttt tcc aac agc aca aat aac ggg tta ttg ttt
    308
   Val Ala Val Leu Pro Phe Ser Asn Ser Thr Asn Asn Gly Leu Leu Phe
   50                  55                  60                  65
   ata aat act act att gcc agc att gct gct aaa gaa gaa ggg gta tcc
    356
   Ile Asn Thr Thr Ile Ala Ser Ile Ala Ala Lys Glu Glu Gly Val Ser
                       70                  75                  80
   atg gct aag aga gaa gaa gct gaa gct gaa gct cca aag ttc gtt aac
    404
   Met Ala Lys Arg Glu Glu Ala Glu Ala Glu Ala Pro Lys Phe Val Asn
                   85                  90                  95
   caa cac ttg tgt ggt tct cac ttg gtt gaa gct ttg tac ttg gtt tgt
    452
   Gln His Leu Cys Gly Ser His Leu Val Glu Ala Leu Tyr Leu Val Cys
   100 105 110
   ggt gaa aga ggt ttc ttc tac act gac aag ggt atc gtt gaa caa tgt
   500
   Gly Glu Arg Gly Phe Phe Tyr Thr Asp Lys Gly Ile Val Glu Gln Cys
   115 120 125
   tgt act tct atc tgt tct ttg tac caa ttg gaa aac tac tgt aac
   545
   Cys Thr Ser Ile Cys Ser Leu Tyr Gln Leu Glu Asn Tyr Cys Asn
   130 135 140
   tagacgcagc ccgcaggctc tagaaactaa gattaatata attatataaa aatat
   600
<210> 5
   <211> 144
   <212> PRT
   <213> Alpha leader fused with N-terminally extended AspB28 IP
<400> 5
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Asp Glu Thr Ala Gln
   20 25 30
   Ile Pro Ala Glu Ala Val Ile Gly Tyr Ser Asp Leu Glu Gly Asp Phe
   35 40 45
   Asp Val Ala Val Leu Pro Phe Ser Asn Ser Thr Asn Asn Gly Leu Leu
   50 55 60
   Phe Ile Asn Thr Thr Ile Ala Ser Ile Ala Ala Lys Glu Glu Gly Val
   65 70 75 80
   Ser Met Ala Lys Arg Glu Glu Ala Glu Ala Glu Ala Pro Lys Phe Val
   85 90 95
   Asn Gln His Leu Cys Gly Ser His Leu Val Glu Ala Leu Tyr Leu Val
   100 105 110
   Cys Gly Glu Arg Gly Phe Phe Tyr Thr Asp Lys Gly Ile Val Glu Gln
           115                     120                 125
   Cys Cys Thr Ser Ile Cys Ser Leu Tyr Gln Leu Glu Asn Tyr Cys Asn
       130                     135                 140
<210> 6
   <211> 600
   <212> DNA
   <213> Alpha leader fused with N-terminally extended AspB28 IP(AspGl yLys)
<220>
   <221> CDS
   <222> (114)..(554)
<400> 6
   tcttgcttaa atctataact acaaaaaaca catacaggaa ttccattcaa gatctgttca
     60
   aacaagaaga ttacaaacta tcaatttcat acacaatata aacgattaaa aga atg
    116
                                                              Met
                                                              1
   aga ttt cct tca att ttt act gca gtt tta ttc gca gca tcc tcc gca
    164
   Arg Phe Pro Ser Ile Phe Thr Ala Val Leu Phe Ala Ala Ser Ser Ala
               5                   10                  15
   tta gct gct cca gtc aac act aca aca gaa gat gaa acg gca caa att
    212
   Leu Ala Ala Pro Val Asn Thr Thr Thr Glu Asp Glu Thr Ala Gln Ile
           20                  25                  30
   ccg gct gaa gct gtc atc ggt tac tca gat tta gaa ggg gat ttc gat
    260
   Pro Ala Glu Ala Val Ile Gly Tyr Ser Asp Leu Glu Gly Asp Phe Asp
       35                  40                  45
   gtt gct gtt ttg cca ttt tcc aac agc aca aat aac ggg tta ttg ttt
    308
   Val Ala Val Leu Pro Phe Ser Asn Ser Thr Asn Asn Gly Leu Leu Phe
   50 55 60 65
   ata aat act act att gcc agc att gct gct aaa gaa gaa ggg gta tcc
   356
   Ile Asn Thr Thr Ile Ala Ser Ile Ala Ala Lys Glu Glu Gly Val Ser
   70 75 80
   atg gct aag aga gaa gaa gct gaa gct gaa gct cca aag ttc gtt aac
   404
   Met Ala Lys Arg Glu Glu Ala Glu Ala Glu Ala Pro Lys Phe Val Asn
   85 90 95
   caa cac ttg tgt ggt tct cac ttg gtt gaa gct ttg tac ttg gtt tgt
   452
   Gln His Leu Cys Gly Ser His Leu Val Glu Ala Leu Tyr Leu Val Cys
   100 105 110
   ggt gaa aga ggt ttc ttc tac act gac aag gat ggg aag ggt atc gtt
   500
   Gly Glu Arg Gly Phe Phe Tyr Thr Asp Lys Asp Gly Lys Gly Ile Val
   115 120 125
   gaa caa tgt tgt act tct atc tgt tct ttg tac caa ttg gaa aac tac
   548
   Glu Gln Cys Cys Thr Ser Ile Cys Ser Leu Tyr Gln Leu Glu Asn Tyr
   130 135 140 145
   tgt aac tagacgcagc ccgcaggctc tagaaactaa gattaatata attata
   600
   Cys Asn
<210> 7
   <211> 147
   <212> PRT
   <213> Alpha leader fused with N-terminally extended AspB28 IP(AspGl yLys)
<400> 7
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Asp Glu Thr Ala Gln
   20 25 30
   Ile Pro Ala Glu Ala Val Ile Gly Tyr Ser Asp Leu Glu Gly Asp Phe
   35 40 45
   Asp Val Ala Val Leu Pro Phe Ser Asn Ser Thr Asn Asn Gly Leu Leu
   50 55 60
   Phe Ile Asn Thr Thr Ile Ala Ser Ile Ala Ala Lys Glu Glu Gly Val
   65 70 75 80
   Ser Met Ala Lys Arg Glu Glu Ala Glu Ala Glu Ala Pro Lys Phe Val
   85 90 95
   Asn Gln His Leu Cys Gly Ser His Leu Val Glu Ala Leu Tyr Leu Val
   100 105 110
   Cys Gly Glu Arg Gly Phe Phe Tyr Thr Asp Lys Asp Gly Lys Gly Ile
   115 120 125
   Val Glu Gln Cys Cys Thr Ser Ile Cys Ser Leu Tyr Gln Leu Glu Asn
   130 135 140
   Tyr Cys Asn
   145
<210> 8
   <211> 6
   <212> PRT
   <213> N-terminal extension
<400> 8
   Glu Glu Gly Glu Pro Lys
   1 5
<210> 9
   <211> 550
   <212> DNA
   <213> TA39 leader fused with N-terminally extended AspB28IP(AspGlyL ys)
<220>
   <221> CDS
   <222> (115)..(489)
<400> 9
   ttcttgctta aatctataac tacaaaaaac acatacagga attccattca agaatagttc
     60
   aaacaagaag attacaaact atcaatttca tacacaatat aaacgattaa aaga atg
    117
                                                                 Met
                                                                 1
   aaa ctg aaa act gta aga tct gcg gtc ctt tcg tca ctc ttt gca tct
    165
   Lys Leu Lys Thr Val Arg Ser Ala Val Leu Ser Ser Leu Phe Ala Ser
               5                   10                  15
   cag gtc ctt ggc caa cca att gac gac act gaa tct aac act act tct
    213
   Gln Val Leu Gly Gln Pro Ile Asp Asp Thr Glu Ser Asn Thr Thr Ser
           20                  25                  30
   gtc aac ttg atg gct gac gac act gaa tct aga ttc gct act aac act
    261
   Val Asn Leu Met Ala Asp Asp Thr Glu Ser Arg Phe Ala Thr Asn Thr
       35                  40                  45
   act ttg gct ggt ggt ttg gat gtt gtt aac ttg atc tcc atg gct aag
    309
   Thr Leu Ala Gly Gly Leu Asp Val Val Asn Leu Ile Ser Met Ala Lys
   50                  55                  60                  65
   aga gaa gaa ggt gaa cca aag ttc gtt aac caa cac ttg tgt ggt tcc
    357
   Arg Glu Glu Gly Glu Pro Lys Phe Val Asn Gln His Leu Cys Gly Ser
                   70                  75                  80
   cac ttg gtt gaa gct ttg tac ttg gtt tgt ggt gaa aga ggt ttc ttc
    405
   His Leu Val Glu Ala Leu Tyr Leu Val Cys Gly Glu Arg Gly Phe Phe
               85                  90                  95
   tac act gac aag gac ggt aag ggt atc gtt gaa caa tgt tgt act tct
   453
   Tyr Thr Asp Lys Asp Gly Lys Gly Ile Val Glu Gln Cys Cys Thr Ser
   100 105 110
   atc tgt tct ttg tac caa ttg gaa aac tac tgt aac tagacgcagc
   499
   Ile Cys Ser Leu Tyr Gln Leu Glu Asn Tyr Cys Asn
   115 120 125
   ccgcaggctc tagaaactaa gattaatata attatataaa aatattatct t
   550
<210> 10
   <211> 125
   <212> PRT
   <213> TA39 leader fused with N-terminally extended AspB28IP(AspGlyL ys)
<400> 10
   Met Lys Leu Lys Thr Val Arg Ser Ala Val Leu Ser Ser Leu Phe Ala
   1 5 10 15
   Ser Gln Val Leu Gly Gln Pro Ile Asp Asp Thr Glu Ser Asn Thr Thr
   20 25 30
   Ser Val Asn Leu Met Ala Asp Asp Thr Glu Ser Arg Phe Ala Thr Asn
   35 40 45
   Thr Thr Leu Ala Gly Gly Leu Asp Val Val Asn Leu Ile Ser Met Ala
   50 55 60
   Lys Arg Glu Glu Gly Glu Pro Lys Phe Val Asn Gln His Leu Cys Gly
   65 70 75 80
   Ser His Leu Val Glu Ala Leu Tyr Leu Val Cys Gly Glu Arg Gly Phe
   85 90 95
   Phe Tyr Thr Asp Lys Asp Gly Lys Gly Ile Val Glu Gln Cys Cys Thr
   100 105 110
   Ser Ile Cys Ser Leu Tyr Gln Leu Glu Asn Tyr Cys Asn
   115 120 125
<210> 11
   <211> 550
   <212> DNA
   <213> TA57 leader fused with N-terminally extended AspB28IP(AspGlyL ys)
<220>
   <221> CDS
   <222> (115)..(486)
<400> 11
   ttcttgctta aatctataac tacaaaaaac acatacagga attccattca agaatagttc
     60
   aaacaagaag attacaaact atcaatttca tacacaatat aaacgattaa aaga atg
    117
                                                                Met
                                                                1
   aaa ctg aaa act gta aga tct gcg gtc ctt tcg tca ctc ttt gca tct
    165
   Lys Leu Lys Thr Val Arg Ser Ala Val Leu Ser Ser Leu Phe Ala Ser
             5                   10                  15
   cag gtc ctt ggc caa cca att gac gac act gaa tct caa act act tct
    213
   Gln Val Leu Gly Gln Pro Ile Asp Asp Thr Glu Ser Gln Thr Thr Ser
         20                  25                  30
   gtc aac ttg atg gct gac gac act gaa tct gct ttc gct act caa act
    261
   Val Asn Leu Met Ala Asp Asp Thr Glu Ser Ala Phe Ala Thr Gln Thr
      35                  40                  45
   aac tct ggt ggt ttg gat gtt gtt ggt ttg atc tcc atg gct aag aga
    309
   Asn Ser Gly Gly Leu Asp Val Val Gly Leu Ile Ser Met Ala Lys Arg
   50                  55                  60                  65
   gaa gaa ggt gaa cca aag ttc gtt aac caa cac ttg tgc ggt tcc cac
    357
   Glu Glu Gly Glu Pro Lys Phe Val Asn Gln His Leu Cys Gly Ser His
                   70                  75                  80
   ttg gtt gaa gct ttg tac ttg gtt tgc ggt gaa aga ggt ttc ttc tac
    405
   Leu Val Glu Ala Leu Tyr Leu Val Cys Gly Glu Arg Gly Phe Phe Tyr
               85                   90                   95
   act gac aag gac ggt aag ggt atc gtt gaa caa tgc tgt acc tcc atc
    453
   Thr Asp Lys Asp Gly Lys Gly Ile Val Glu Gln Cys Cys Thr Ser Ile
           100                  105                  110
   tgc tcc ttg tac caa ttg gaa aac tac tgc aac tagacgcagc ccgcaggctc
    506
   Cys Ser Leu Tyr Gln Leu Glu Asn Tyr Cys Asn
       115                  120
   tagaaactaa gattaatata attatataaa aatattatct tctt
    550
<210> 12
   <211> 124
   <212> PRT
   <213> TA57 leader fused with N-terminally extended AspB28IP(AspGlyL ys)
<400> 12
   Met Lys Leu Lys Thr Val Arg Ser Ala Val Leu Ser Ser Leu Phe Ala
   1               5                   10                  15
   Ser Gln Val Leu Gly Gln Pro Ile Asp Asp Thr Glu Ser Gln Thr Thr
               20                  25                  30
   Ser Val Asn Leu Met Ala Asp Asp Thr Glu Ser Ala Phe Ala Thr Gln
           35                  40                  45
   Thr Asn Ser Gly Gly Leu Asp Val Val Gly Leu Ile Ser Met Ala Lys
       50                  55                  60
   Arg Glu Glu Gly Glu Pro Lys Phe Val Asn Gln His Leu Cys Gly Ser
   65                  70                  75                  80
   His Leu Val Glu Ala Leu Tyr Leu Val Cys Gly Glu Arg Gly Phe Phe
                   85                  90                  95
   Tyr Thr Asp Lys Asp Gly Lys Gly Ile Val Glu Gln Cys Cys Thr Ser
   100 105 110
   Ile Cys Ser Leu Tyr Gln Leu Glu Asn Tyr Cys Asn
   115 120
<210> 13
   <211> 27
   <212> DNA
   <213> Primer
<400> 13
   taaatctata actacaaaaa acacata
   27
<210> 14
   <211> 109
   <212> DNA
   <213> Primer
<220>
   <221> misc_feature
   <222> (22) .. (24)
   <223> n is a, t, g or c and m is c or a
<400> 14
   ccaaagaaga tgtgactgtt cnnmcccttc ccatagcaac ttgttacaac atgaagatag
   60
   acaagaaaca tggttaacct tttgatgaca ttgatcagat ctttgattc
   109
<210> 15
   <211> 45
   <212> PRT
   <213> TA39 Synthetic propeptide
<400> 15
   Gln Pro Ile Asp Asp Thr Glu Ser Asn Thr Thr Ser Val Asn Leu Met
   1 5 10 15
   Ala Asp Asp Thr Glu Ser Arg Phe Ala Thr Asn Thr Thr Leu Ala Gly
   20 25 30
   Gly Leu Asp Val Val Asn Leu Ile Ser Met Ala Lys Arg
   35 40 45
<211> 44
   <212> PRT
   <213> TA57 Synthetic propeptide
<400> 16